(19) Europäisches Patentamt
European Patent Office
Office européen des brevets

(11) EP 1 733 210 B1

(12) # FASCICULE DE BREVET EUROPEEN

(45) Date de publication et mention de la délivrance du brevet:
**22.03.2017 Bulletin 2017/12**

(21) Numéro de dépôt: **05746923.1**

(22) Date de dépôt: **08.04.2005**

(51) Int Cl.:
***G01N 21/64*** (2006.01) ***G01N 33/22*** (2006.01)
***C09K 11/06*** (2006.01) ***G01N 33/00*** (2006.01)

(86) Numéro de dépôt international:
**PCT/FR2005/050222**

(87) Numéro de publication internationale:
**WO 2005/103653 (03.11.2005 Gazette 2005/44)**

(54) **Capteurs chimiques comprenant des polymères conjugués fluorescents comme matériaux sensibles, et leur utilisation pour la détection ou le dosage de composés nitrés**

Chemischer Sensor auf der Basis von konjugierten, fluoreszierenden Polymeren als Sensor-Materialien, und deren Verwendung für den Nachweis oder die Dosierung von Nitroverbindungen

Chemical sensors comprising fluorescent conjugated polymers as sensitive materials, and the use thereof for the detection or dosage of nitrated compounds

(84) Etats contractants désignés:
**AT BE BG CH CY CZ DE DK EE ES FI FR GB GR HU IE IS IT LI LT LU MC NL PL PT RO SE SI SK TR**

(30) Priorité: **09.04.2004 FR 0450719**

(43) Date de publication de la demande:
**20.12.2006 Bulletin 2006/51**

(73) Titulaire: **Commissariat à l'Énergie Atomique et aux Énergies Alternatives**
**75015 Paris (FR)**

(72) Inventeurs:
- **HAIRAULT, Lionel**
  **F-37150 BLERE (FR)**
- **PASQUINET, Eric**
  **F-37550 Saint Avertin (FR)**
- **MONTMEAT, Pierre**
  **F-37520 La Riche (FR)**
- **MOREAU, Joël**
  **F-34000 Montpellier (FR)**
- **LERE-PORTE, Jean-Pierre**
  **F-34090 Montpellier (FR)**
- **WAKIM, Salem**
  **Gatineau, Québec J8Y 2S9 (CA)**
- **SEREIN-SPIRAU, Françoise**
  **F-34090 Montpellier (FR)**

(74) Mandataire: **Ahner, Philippe et al**
**BREVALEX**
**95, rue d'Amsterdam**
**75378 Paris Cedex 8 (FR)**

(56) Documents cités:
**EP-A- 0 794 428 EP-A- 1 281 744**

- **LÈRE-PORTE J-P; MOREAU J J E; SEREIN-SPIRAU F; WAKIM S;: "New chiral pi-conjugated polymers based on a (1R,2R)-diiminocyclohexane chiral unit with weak interchain pi stacking" CHEMICAL COMMUNICATIONS, vol. 24, 21 décembre 2002 (2002-12-21), pages 3020-3021, XP002298566 CAMBRIDGE, ENGLAND cité dans la demande**
- **LÈRE-PORTE J-P; MOREAU J J E; SEREIN-SPIRAU F; WAKIM S: "A chiral polymer with alternating conjugated segments and (1R,2R)-1,2-diaminocyclohexane as a unit with C2 symmetry" TETRAHEDRON LETTERS, vol. 42, no. 17, 23 avril 2001 (2001-04-23), pages 3073-3076, XP002298567 UNITED KINGDOM cité dans la demande**
- **HUANG H-M; WANG K-M; HUANG S-S; ZHOU L-J; LI D: "Optical membrane for o-nitroaniline based on fluorescence energy transfer between a small molecule and a conjugated polymer" ANALYTICA CHIMICA ACTA, vol. 481, no. 1, 20 mars 2003 (2003-03-20), pages 109-117, XP002298568 NETHERLANDS**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- MIAO YI-JUN ET AL: "Fluorescence sensory polymers containing rigid non-planar aromatic scaffolds" PAPERS PRESENTED AT THE MEETING - AMERICAN CHEMICAL SOCIETY. DIVISION OF POLYMER CHEMISTRY, vol. 39, no. 2, août 1998 (1998-08), pages 1081-1082, XP002111384 ISSN: 0032-3934
- YANG ET AL: "Fluorescent Porous Polymer Films as TNT Chemosensors: Electronic and Structural Effects" JOURNAL OF THE AMERICAN CHEMICAL SOCIETY, AMERICAN CHEMICAL SOCIETY, WASHINGTON, DC, US, vol. 120, no. 46, 1998, pages 11864-11873, XP002111385 ISSN: 0002-7863
- TANESE M C ET AL: "Poly(phenyleneethynylene) polymers bearing glucose substituents as promising active layers in enantioselective chemiresistors" SENS ACTUATORS, B CHEM; SENSORS AND ACTUATORS, B: CHEMICAL JUN 1 2004, vol. 100, no. 1-2, 10 juin 2003 (2003-06-10), pages 17-21, XP002346790

## Description

### DOMAINE TECHNIQUE

La présente invention se rapporte à l'utilisation de capteurs chimiques comprenant des polymères conjugués fluorescents en tant que matériaux sensibles, pour détecter ou doser des composés nitrés, en particulier des composés nitroaromatiques tels que le nitrobenzène (NB), le dinitrobenzène (DNB), le trinitrobenzène (TNB), le nitrotoluène (NT), le dinitrotoluène (DNT), le 2,4,6-trinitrotoluène (TNT) et analogues, ainsi qu'à des capteurs chimiques particuliers.

De tels capteurs sont utiles pour la détection d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Ils sont également utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant, stockant et/ou manipulant des composés nitrés.

### ÉTAT DE LA TECHNIQUE ANTÉRIEURE

La détection d'explosifs est un problème d'intérêt crucial, notamment en matière de sécurité civile.

A l'heure actuelle, plusieurs méthodes sont utilisées pour détecter des vapeurs de composés nitrés entrant dans la constitution des explosifs, comme l'emploi de chiens *"renifleurs"* dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens *"renifleurs"* présente l'inconvénient de nécessiter une longue formation des chiens et de leurs maîtres et d'être inadaptée à des opérations prolongées car la durée d'attention des chiens est limitée.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques est en plein essor. Le fonctionnement de ces capteurs est basé sur l'utilisation d'un film d'un matériau sensible, c'est-à-dire d'un matériau dont au moins une propriété physique est modifiée au contact des molécules recherchées, qui revêt un système apte à mesurer en temps réel toute variation de cette propriété physique et de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

Pour la détection de composés nitrés et, en particulier, de composés nitroaromatiques, différentes familles de composés ont été proposées pour servir de matériaux sensibles dans des capteurs, dont des polysiloxanes, des polyéthylène glycols, des amines, des phtalocyanines, des cyclodextrines, des adsorbants tels que le charbon, et des composés fluorescents.

Concernant ces derniers, les travaux effectués à ce jour s'inscrivent essentiellement dans deux axes de recherche différents, à savoir, d'une part, la mise au point de capteurs à base de silicium poreux nanostructuré (M.J. Sailor et al., SPIE Proceeding, The International Society of Optical Engineering, 3713, 1999, 54-65 **[1]**) ou de silice associée à un colorant organique (K.J. Albert et D.R. Walt, Anal. Chem., 72, 2000, 1947 **[2]**) et, d'autre part, la réalisation de capteurs utilisant des molécules organiques à forte conjugaison.

On sait notamment que les polymères $\pi$-conjugués présentent, d'une manière générale, une grande sensibilité vis-à-vis des composés nitrés et, notamment, des composés nitroaromatiques.

Toutefois, pour pouvoir servir de matériaux sensibles dans des capteurs chimiques, il convient que ces polymères $\pi$-conjugués présentent, de plus, d'excellentes propriétés de perméabilité, d'affinité chimique et de sélectivité vis-à-vis des composés nitrés, ainsi qu'un rendement de fluorescence élevé. Il convient, en outre, que ces propriétés soient stables dans le temps de manière à conférer aux capteurs une durée de vie satisfaisante, c'est-à-dire en pratique de plusieurs semaines, voire de plusieurs mois.

La présente invention vise justement à fournir un capteur chimique qui comprend des polymères $\pi$-conjugués

fluorescents satisfaisant à tous ces critères, en tant que matériaux sensibles.

## EXPOSÉ DE L'INVENTION

La présente invention a donc, en premier lieu, pour objet l'utilisation d'un capteur chimique qui comprend, en tant que matériau sensible, au moins un polymère comprenant au moins un motif répétitif répondant à la formule générale (I) ci-après :

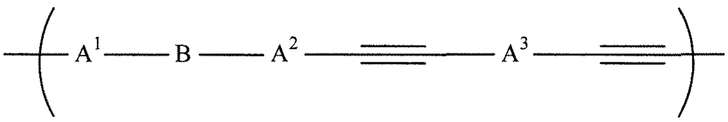

(I)

dans laquelle :

$A^1$, $A^2$ et $A^3$ représentent, indépendamment les uns des autres, un groupe phényle ou thiényle substitué ou non substitué ; et
B représente un groupe chiral de symétrie C2, substitué ou non substitué, ou bien un groupe répondant à l'une des formules (a), (b) et (c) ci-après :

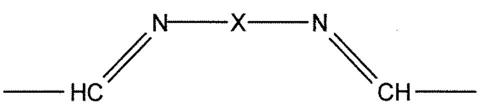

(a)

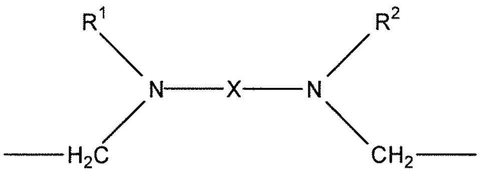

(b)

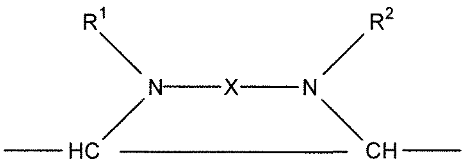

(c)

où :

X représente un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ;
$R^1$ et $R^2$ représentent indépendamment l'un de l'autre :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore
- une fonction chimique comprenant au moins un hétéroatome ;

pour la détection ou le dosage d'un ou plusieurs composés nitrés.

Dans ce qui précède et ce qui suit, on entend par *"hétéroatome",* tout atome autre que de carbone ou d'hydrogène comme, par exemple, un atome d'oxygène, de soufre, d'azote, de fluor, de chlore, de phosphore, de bore ou encore de silicium, les atomes d'oxygène, de soufre et d'azote étant préférés.

On entend par *"fonction chimique comprenant au moins un hétéroatome",* toute fonction chimique comportant un ou plusieurs atomes autres que de carbone ou d'hydrogène et, notamment, une fonction chimique comportant un ou plusieurs atomes d'oxygène, de soufre, d'azote et/ou d'halogène. Cette fonction chimique peut, en particulier, être choisie parmi les fonctions -COOH, $-COOR^3$, -CHO, -CO-, -OH, $-OR^3$, -SH, -SR, $-SO_2R^3$, $-NH_2$, $-NHR^3$, $-NR^3R^4$, $-CONH_2$, $-CONHR^3$, $-CONR^3R^4$, $-C(Hal)_3$, $-OC(Hal)_3$, -C(O)Hal, -CN, -COOCHO, $-COOCOR^3$ et phénol, dans lesquelles :

- $R^3$ représente un groupe hydrocarboné, linéaire, ramifié ou cyclique, saturé ou insaturé, comprenant de 1 à 100 atomes de carbone, ou une liaison covalente dans le cas où ladite fonction chimique forme pont dans un groupe hydrocarboné en $C_2$ à $C_{100}$ ;
- $R^4$ représente un groupe hydrocarboné, linéaire, ramifié ou cyclique, saturé ou insaturé, comprenant de 1 à 100 atomes de carbone, ce groupe pouvant être identique ou différent du groupe hydrocarboné représenté par $R^3$ ; tandis que
- Hal représente un atome d'halogène, par exemple un atome de fluor, de chlore ou de brome.

On entend, par ailleurs, par "*groupe aromatique",* tout groupe mono- ou polycyclique satisfaisant à la règle de Hückel, c'est-à-dire présentant un nombre d'électrons $\pi$ délocalisés égal à (4n + 2) et, par *"groupe hétéroaromatique",* tout groupe mono- ou polycyclique tel qu'il vient d'être défini, mais comprenant, dans le cycle ou au moins l'un des cycles qui le constituent, un ou plusieurs hétéroatomes. A titre d'exemples de groupe aromatique susceptible d'être utilisé, on peut citer les groupes cyclopentadiényle, phényle, benzyle, biphényle, phényl-acétylényle, pyrényle ou anthracényle, tandis qu'à titre d'exemples de groupe hétéroaromatique, on peut citer les groupes furanyle, pyrrolyle, thiényle, oxazolyle, pyrazolyle, thiazolyle, imidazolyle, triazolyle, pyridinyle, pyranyle, quinoléinyle, pyrazinyle et pyrimidinyle. Conformément à l'invention, ce groupe aromatique ou hétéroaromatique peut être substitué, notamment par une ou plusieurs fonctions chimiques comprenant au moins un hétéroatome, telles que celles mentionnées ci-dessus.

Selon une première disposition préférée de l'invention, dans la formule générale (I), $A^1$, $A^2$ et $A^3$ sont chacun un groupe phényle, auquel cas le motif répétitif de formule générale (I) répond, de préférence, à la formule particulière (I-A) ci-après :

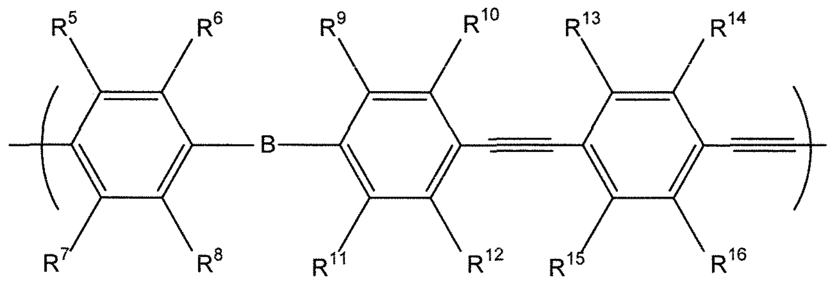

(I-A)

dans laquelle :

$R^5$, $R^6$, $R^7$, $R^8$, $R^9$, $R^{10}$, $R^{11}$, $R^{12}$, $R^{13}$, $R^{14}$, $R^{15}$ et $R^{16}$ représentent indépendamment les uns des autres :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore

- une fonction chimique comprenant au moins un hétéroatome ; et

B a la même signification que précédemment.

Selon une autre disposition préférée de l'invention, dans la formule générale (I), $A^1$ et $A^2$ sont chacun un groupe thiényle, tandis que $A^3$ est un groupe phényle, auquel cas le motif répétitif de formule générale (I) répond, de préférence, à la formule particulière (I-B) ci-après :

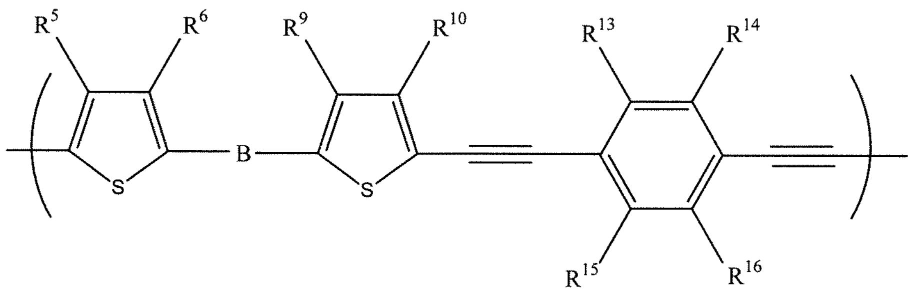

(I-B)

dans laquelle :

$R^5$, $R^6$, $R^9$, $R^{10}$, $R^{13}$, $R^{14}$, $R^{15}$ et $R^{16}$ représentent indépendamment les uns des autres :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore
- une fonction chimique comprenant au moins un hétéroatome ; et

B a la même signification que précédemment.

Toutefois, des motifs répétitifs autres que ceux répondant aux formules particulières (I-A) et (I-B) peuvent également être envisagés comme, par exemple, des motifs répétitifs de formule générale (I) dans laquelle $A^1$, $A^2$ et $A^3$ sont chacun un groupe thiényle.

On notera que, lorsque $R^5$, $R^6$, $R^7$, $R^8$, $R^9$, $R^{10}$, $R^{11}$, $R^{12}$, $R^{13}$, $R^{14}$, $R^{15}$ et/ou $R^{16}$ représentent, dans les formules particulières (I-A) et (I-B), un groupe hydrocarboné comprenant deux atomes de carbone ou plus et que ce groupe comporte un ou plusieurs hétéroatomes et/ou une ou plusieurs fonctions chimiques et/ou un ou plusieurs groupes aromatiques ou hétéroaromatiques, alors cet(ces) hétéroatome(s), cette(ces) fonction(s) chimique(s) et ce(s) groupe(s) aromatique(s) ou hétéroaromatique(s) peuvent aussi bien former pont à l'intérieur de ce groupe hydrocarboné qu'être portés latéralement par lui ou encore se situer à son extrémité.

De façon similaire, lorsque X, $R^1$ et/ou $R^2$ représentent, dans les formules (a), (b) et (c), un groupe hydrocarboné comprenant deux atomes de carbone ou plus et que ce groupe comporte un ou plusieurs hétéroatomes et/ou une ou plusieurs fonctions chimiques et/ou un ou plusieurs groupes aromatiques ou hétéroaromatiques, alors cet(ces) hétéroatome(s), cette(ces) fonction(s) chimique(s) et ce(s) groupe(s) aromatique(s) ou hétéroaromatique(s) peuvent aussi bien former pont à l'intérieur de ce groupe hydrocarboné, être portés latéralement par lui ou encore se situer à son extrémité.

On notera également que, si les groupes hydrocarbonés susceptibles d'être représentés par $R^5$, $R^6$, $R^7$, $R^8$, $R^9$, $R^{10}$, $R^{11}$, $R^{12}$, $R^{13}$, $R^{14}$, $R^{15}$ et/ou $R^{16}$ dans les formules particulières (I-A) et (I-B) et par X, $R^1$ et/ou $R^2$ dans les formules (a), (b) et (c) peuvent comprendre jusqu'à 100 atomes de carbone, on préfère généralement qu'ils ne comprennent pas plus de 50 atomes de carbone et, mieux encore, pas plus de 30 atomes de carbone. Ceci est également vrai pour tous les autres groupes hydrocarbonés en $C_1$ à $C_{100}$ envisagés dans le cadre de la présente invention.

Comme précédemment mentionné, B peut représenter, dans la formule générale (I), un groupe chiral de symétrie C2, auquel cas il peut notamment être :

* un groupe hydrocarboné aliphatique comme, par exemple, un groupe répondant à la formule (i) ci-après :

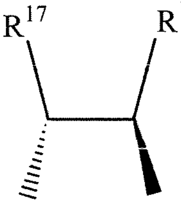

(i)

dans laquelle $R^{17}$ représente un atome d'halogène ; un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome ;
* un groupe cyclique à cycle saturé comme, par exemple, un groupe répondant à l'une des formules (ii), (iii) et (iv) ci-après :

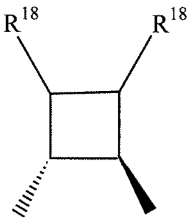

(ii)

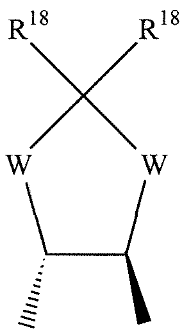

(iii)

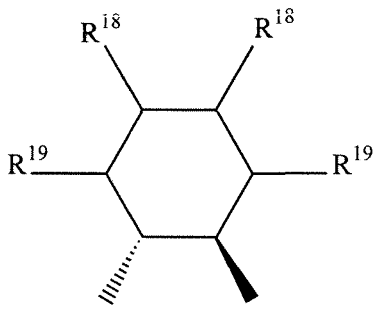

(iv)

dans lesquelles :

- $R^{18}$ et $R^{19}$ représentent, indépendamment l'un de l'autre, un atome d'hydrogène ou d'halogène ; un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome ;

- W représente un atome d'oxygène, un groupe -NH ou un groupe $-CH_2$, éventuellement substitué par un groupe hydrocarboné linaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome ;

* un groupe aromatique comme, par exemple, un groupe dérivé du 1,1'-binaphtyle, répondant à l'une des formules (v), (vi) et (vii) ci-après :

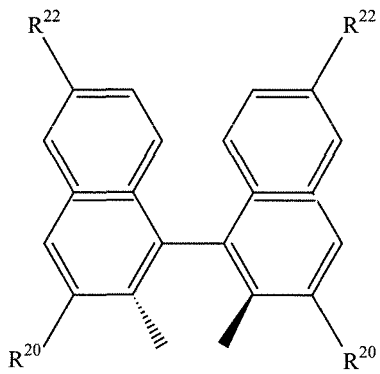

(v)

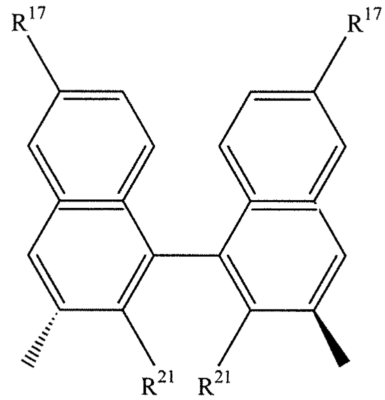

(vi)

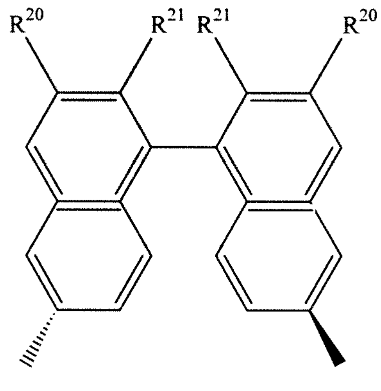

(vii)

dans lesquelles $R^{20}$, $R^{21}$ et $R^{22}$ représentent, indépendamment les uns des autres, un atome d'hydrogène ou d'halogène, un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs

fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome.

Toutefois, on préfère que B soit un groupe répondant à l'une des formules (a), (b) et (c) telles que précédemment définies, et notamment que B soit un groupe de formule (a), (b) ou (c) dans laquelle X est un groupe hydrocarboné chiral de symétrie C2, l'association entre la fonctionnalité diimine ou diamine présente dans ces groupes et une chiralité de symétrie de type C2 s'étant, en effet, révélée conduire à une optimisation à la fois de l'affinité chimique du polymère vis-à-vis des composés nitrés, de son rendement de fluorescence et de la porosité des films minces ou des monolithes préparés à partir de ce polymère.

En particulier, on préfère les polymères comprenant au moins un motif répétitif de formule générale (I) dans laquelle B représente un groupe répondant à l'une des formules (a), (b) et (c) où X est un groupe hydrocarboné chiral de symétrie C2 choisi parmi les groupes répondant aux formules (i) à (vii) telles que précédemment définies.

Parmi ces polymères, on préfère tout particulièrement :

- ceux qui comprennent au moins un motif répétitif répondant à l'une des formules particulières (I-A-a), (I-A-b) et (I-A-c) ci-après :

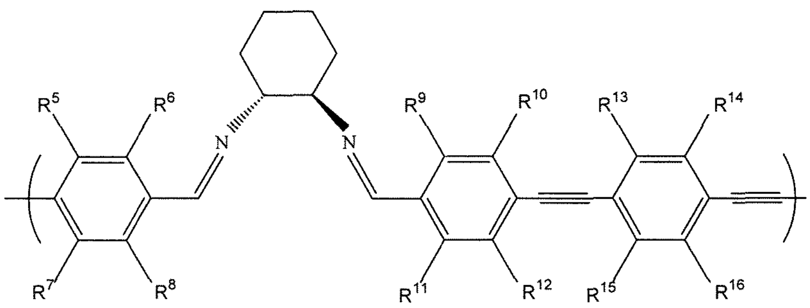

(I-A-a)

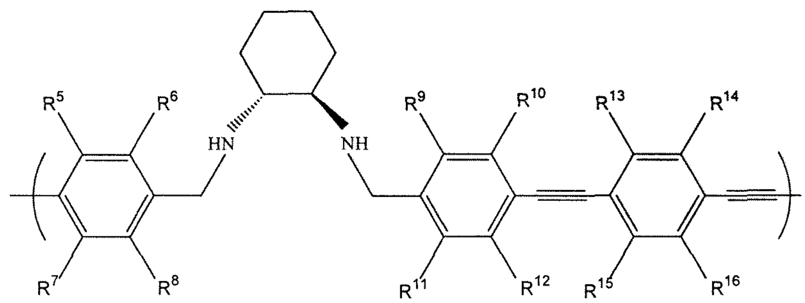

(I-A-b)

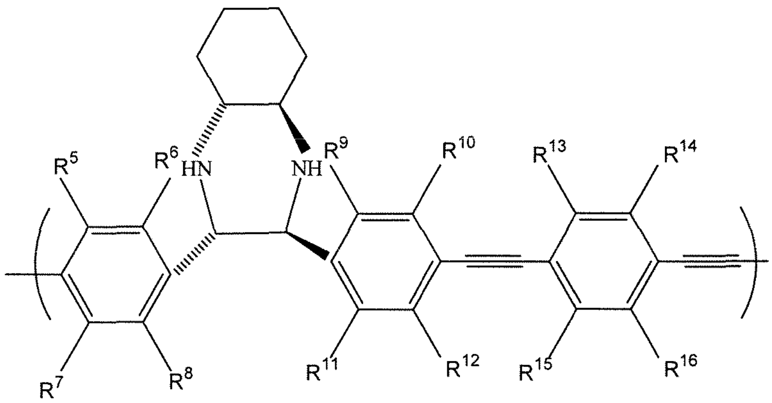

(I-A-c)

dans lesquelles $R^5$ à $R^{16}$ sont tels que précédemment définis ; et

- ceux qui comprennent au moins un motif répétitif répondant à l'une des formules particulières (I-B-a), (I-B-b) et (I-B-c) ci-après :

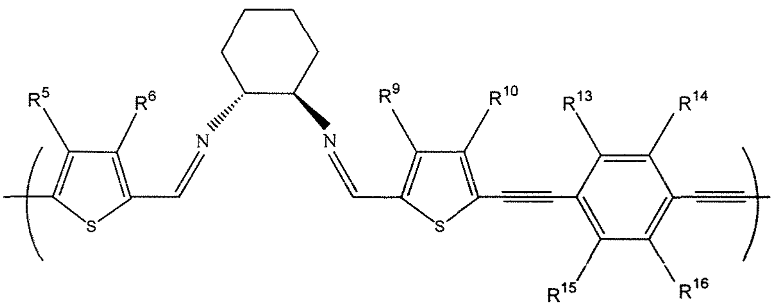

(I-B-a)

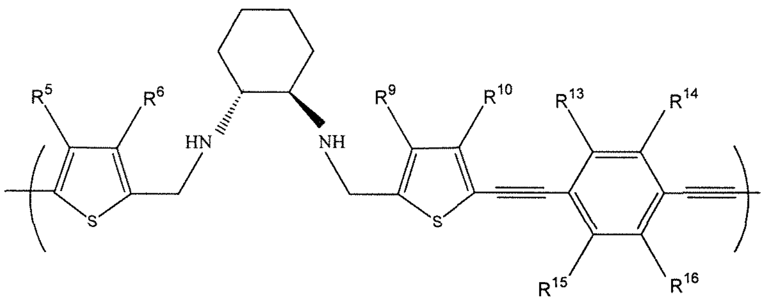

(I-B-b)

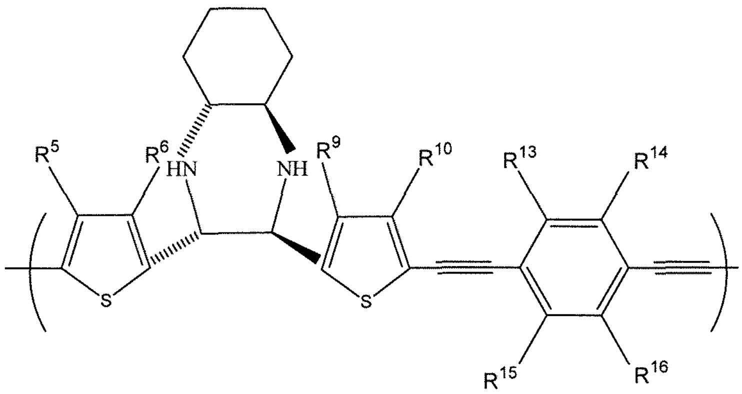

(I-B-c)

dans lesquelles $R^5$, $R^6$, $R^9$, $R^{10}$ et $R^{13}$ à $R^{16}$ sont tels que précédemment définis.

Selon encore une autre disposition préférée de l'invention, dans la formule particulière (I-A), au moins l'un de $R^5$ à $R^{16}$ représente un groupe alcoxy, linéaire ou ramifié, en $C_1$ à $C_{20}$, celui ou ceux de $R^5$ à $R^{16}$ qui ne représentent pas un groupe alcoxy, s'il y en a, représentant alors, de préférence, un atome d'hydrogène.

De manière similaire, on préfère que, dans la formule particulière (I-B), au moins l'un de $R^5$, $R^6$, $R^9$, $R^{10}$ et de $R^{13}$ à $R^{16}$ représente un groupe alcoxy, linéaire ou ramifié, en $C_1$ à $C_{20}$ et, mieux encore, en $C_5$ à $C_{10}$, celui celui ou ceux de $R^5$, $R^6$, $R^9$, $R^{10}$ et de $R^{13}$ à $R^{16}$ qui ne représentent pas un groupe alcoxy, s'il y en a, représentant alors un atome d'hydrogène.

Il est ainsi possible, en jouant sur le nombre de groupes alcoxy portés par les groupes phényles et/ou thiényles ainsi que sur le nombre d'atomes de carbone présents dans ces groupes alcoxy de moduler la solubilité du polymère dans les solvants organiques.

Selon une disposition particulièrement préférée de l'invention, le polymère comprend au moins un motif répétitif de formule particulière (I-A-a), (I-A-b) ou (I-A-c) dans laquelle $R^{14}$ et $R^{15}$ représentent un groupe alcoxy, linéaire ou ramifié, en $C_1$ à $C_{20}$ et, mieux encore, en $C_5$ à $C_{10}$, tandis que $R^5$ à $R^{13}$ et $R^{16}$ représentent un atome d'hydrogène.

Un tel polymère peut notamment être un polymère ayant pour motif répétitif, le motif de formule particulière (I-A-a) ou (I-A-b) dans laquelle $R^{14}$ et $R^{15}$ représentent un groupe octoxy, et $R^5$ à $R^{13}$ et $R^{16}$ représentent un atome d'hydrogène.

Selon une autre disposition particulièrement préférée de l'invention, le polymère comprend au moins un motif répétitif de formule particulière (I-B-a), (I-B-b) ou (I-B-c) dans laquelle $R^{14}$ et $R^{15}$ représentent un groupe alcoxy, linéaire ou ramifié, en $C_1$ à $C_{20}$ et, mieux encore, en $C_5$ à $C_{10}$, tandis que $R^5$, $R^6$, $R^9$, $R^{10}$, $R^{13}$ et $R^{16}$ représentent un atome d'hydrogène.

Un tel polymère peut notamment être un polymère ayant pour motif répétitif, le motif répétitif de formule particulière (I-B-a) dans laquelle $R^{14}$ et $R^{15}$ représentent un groupe octoxy, tandis que $R^5$, $R^6$, $R^9$, $R^{10}$, $R^{13}$ et $R^{16}$ représentent un atome d'hydrogène.

Conformément à l'invention, le polymère est, de préférence, un homopolymère, c'est-à-dire qu'il est constitué par la répétition d'un seul motif répétitif de formule générale (I), ce motif pouvant être répété jusqu'à 10000 fois.

Toutefois, en variante, le polymère peut également être un copolymère, auquel cas il peut aussi bien être constitué par différents motifs répétitifs répondant tous à la formule générale (I), que comprendre un ou plusieurs motifs répétitifs de formule générale (I) et un ou plusieurs motifs répétitifs ne répondant pas à cette formule.

En effet, il peut, par exemple, être utile d'inclure dans le polymère des motifs répétitifs issus d'un monomère du type siloxane en vue d'augmenter encore sa tenue au vieillissement, ou d'un monomère du type diéthynyl dibromobenzène ou diéthynyl diiodobenzène pour lui conférer une résistance mécanique supérieure.

Selon encore une autre disposition préférée de l'invention, le polymère est présent dans le capteur sous la forme d'un film mince qui recouvre l'une ou les deux faces d'un substrat, ce film mince ayant une épaisseur allant de 10 angströms à 100 microns.

Il peut, toutefois, également être présent sous la forme d'un monolithe comme, par exemple, un cylindre présentant une certaine porosité de sorte à rendre accessible aux composés à détecter ou à doser l'ensemble des molécules formant ledit polymère.

Un tel film peut être obtenu par l'une quelconque des techniques proposées à ce jour pour réaliser un film mince sur la surface d'un substrat, par exemple :

* par pulvérisation, par dépôt à la tournette ("*spin coating*") ou par dépôt à la goutte ("drop *coating*") sur le substrat d'une solution contenant le polymère ou le composite ;
* par trempage-retrait ("*dip coating*") du substrat dans une solution contenant le polymère ou le composite ;
* par la technique de Langmuir-Blodgett ;
* par dépôt électrochimique ; ou encore
* par polymérisation *in situ,* c'est-à-dire directement sur la surface du substrat, d'un monomère précurseur du polymère.

Le substrat ainsi que le système de mesure du capteur sont choisis en fonction de la propriété physique du polymère dont les variations induites par la présence des composés à détecter ou à doser sont destinées à être mesurées par le capteur.

En l'espèce, les variations de deux propriétés physiques se sont révélées particulièrement intéressantes à mesurer : il s'agit, d'une part, des variations de l'intensité de la fluorescence émise par le polymère et, d'autre part, des variations de la masse de ce polymère.

Aussi, le capteur est-il, de préférence, un capteur optique dont le fonctionnement est basé sur la mesure des variations de l'intensité de la fluorescence émise par le polymère, ou un capteur gravimétrique dont le fonctionnement est basé sur la mesure des variations de la masse du polymère.

Le principe de fonctionnement des capteurs optiques à base de fluorescence a notamment été décrit par B. Valeur dans Molecular Fluorescence : Principles and Applications, 2002, Ed. WILEY VCH, New York **[3].** Généralement, ces capteurs comprennent un substrat en verre de qualité optique dont l'une des faces est recouverte d'un film mince du matériau sensible. L'intensité de la fluorescence émise par le matériau sensible peut être mesurée sur l'ensemble du spectre d'émission de ce matériau. Toutefois, il est préférable d'effectuer les mesures d'intensité de fluorescence à la longueur d'onde d'émission donnant les valeurs d'intensité maximales pour la longueur d'onde d'excitation conduisant, elle, au meilleur rapport signal/bruit pour l'acquisition des intensités de fluorescence.

A titre d'exemples de capteurs gravimétriques, on peut citer les capteurs du type à microbalance à quartz, les capteurs à ondes de surface, plus connus sous l'acronyme anglo-saxon *SAW* ("*Surface Acoustic Wave*"), tels que les capteurs à ondes de Love et les capteurs à ondes de Lamb, ainsi que les microleviers.

Parmi les capteurs gravimétriques, on préfère plus particulièrement les capteurs du type microbalance à quartz. Ce type de capteurs, dont le principe de fonctionnement a été décrit par J.A.O. Sanchez-Pedrono et al. dans Anal. Chem. Acta, 182, 1986, 285 **[4],** comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du substrat.

Bien entendu, il est également possible d'utiliser un polymère tel que précédemment défini, comme matériau sensible dans des capteurs conçus pour mesurer des variations d'une propriété physique autre que l'intensité de fluorescence et la masse comme, par exemple, des variations d'une propriété électrique telle que la conductivité, ou d'une propriété optique autre que la fluorescence comme, par exemple, l'absorbance.

Conformément à l'invention, le capteur peut être de type multicapteur, c'est-à-dire qu'il peut être constitué par plusieurs capteurs élémentaires comprenant des matériaux sensibles différents les uns des autres, ou munis de substrats et de systèmes de mesure différents les uns des autres comme, par exemple, un ou plusieurs capteurs à base de fluorescence et/ou un ou plusieurs capteurs gravimétriques, l'essentiel étant que l'un au moins de ces capteurs élémentaires comprenne un polymère tel que précédemment défini, en tant que matériau sensible.

Des capteurs comportant un polymère tel que précédemment défini, en tant que matériau sensible, se sont révélés présenter de nombreux avantages, notamment :

* une aptitude à détecter spécifiquement les composés nitrés, et en particulier les composés nitroaromatiques, avec une grande sensibilité puisqu'ils sont capables de détecter leur présence à des concentrations inférieures au ppm (partie par million) et même au dixième de ppm, doublée d'une spécificité vis-à-vis de ces composés,
* une rapidité de réponse et une reproductibilité de cette réponse,
* une stabilité des performances dans le temps et, partant, une durée de vie très satisfaisante,
* une aptitude à fonctionner en continu,
* un coût de fabrication compatible avec une production de capteurs en série, une très faible quantité de polymère (c'est-à-dire en pratique de quelques mg) étant nécessaire pour la fabrication d'un capteur, et
* la possibilité d'être miniaturisés et, partant, d'être aisément transportables et manipulables sur tout type de sites.

Les composés nitrés peuvent aussi bien se présenter sous forme solide, liquide ou gazeuse (vapeurs), mais étant, de préférence, sous forme gazeuse.

Conformément à l'invention, le ou les composés nitrés destinés à être détectés ou dosés sont choisis parmi

les composés nitroaromatiques, les nitramines, les nitrosamines et les esters nitriques.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'amino-dinitrotoluène, le dinitrotrifluorométhylbenzène, le chlorodinitrotrifluorométhylbenzène, l'hexanitrostilbène ou encore le trinitrophénol (ou acide picrique).

Les nitramines sont, elles, par exemple la cyclotétraméthylènetétranitramine (ou octogène), la cyclotriméthylènetrinitramine (ou hexogène) et la trinitrophénylméthylnitramine (ou tétryle), tandis que les nitrosamines sont, par exemple, la nitrosodiméthylamine.

Quant aux esters nitriques, il s'agit, par exemple, de pentrite, de dinitrate d'éthylène glycol, de dinitrate de diéthylène glycol, de nitroglycérine ou de nitroguanidine.

Selon encore une autre disposition préférée de l'invention, le capteur est utilisé pour la détection ou le dosage d'explosifs.

L'invention a également pour objet un capteur chimique tel que défini dans les revendications 23 à 28.

D'autres caractéristiques et avantages de l'invention apparaîtront mieux à la lecture du complément de description qui suit, qui se rapporte à des exemples de capteurs et de mise en évidence de leurs propriétés, et qui se réfère aux dessins annexés.

Bien entendu, ces exemples ne sont donnés qu'à titre d'illustrations de l'objet de l'invention et ne constituent en aucun cas une limitation de cet objet.

## BRÈVE DESCRIPTION DES DESSINS

La figure 1 représente l'évolution de l'intensité de la fluorescence émise par un premier exemple de capteur ($\lambda_{émission}$ : 507 nm ; $\lambda_{excitation}$ : 404 nm) lorsque ce capteur est exposé alternativement à de l'azote pur et à des mélanges d'azote et de 2,4-dinitrotrifluorométhoxy-benzène (DNTFMB).

La figure 2 représente l'évolution de l'intensité de la fluorescence émise par le premier exemple de capteur ($\lambda_{émission}$ : 507 nm ; $\lambda_{excitation}$ : 404 nm) lorsque ce capteur est exposé alternativement à de l'azote pur et à des mélanges d'azote et respectivement de DNTFMB, de dichlorométhane, de cyclohexane, de méthyléthylcétone, de toluène, de méthylisobutylcétone, d'acétate d'éthyle, et à nouveau de DNTFMB.

La figure 3 représente la variation de l'intensité de la fluorescence émise par le premier exemple de capteur ($\lambda_{émission}$ : 507 nm ; $\lambda_{excitation}$ : 404 nm) lorsque ce capteur est soumis à une série d'expositions à un mélange d'azote et de DNTFMB de 10 minutes chacune, réparties sur une période de 96 jours.

La figure 4 représente l'évolution de l'intensité de la fluorescence émise par un deuxième exemple de capteur ($\lambda_{émission}$ : 500 nm ; $\lambda_{excitation}$ = 397 nm) lorsque ce capteur est exposé alternativement à de l'azote pur et à des mélanges d'azote et de DNTFMB.

La figure 5 représente l'évolution de la fréquence de vibration du quartz d'un troisième exemple de capteur lorsque ce capteur est exposé alternativement à l'air ambiant et à des mélanges d'air ambiant et de vapeurs de DNTFMB.

La figure 6 représente l'évolution de l'intensité de la fluorescence émise par un quatrième exemple de capteur ($\lambda_{émission}$ : 491 nm ; $\lambda_{excitation}$ = 397 nm) lorsque ce capteur est exposé alternativement à de l'azote pur et à un mélange d'azote et de DNTFMB.

## EXPOSÉ DÉTAILLÉ DE MODES DE RÉALISATION PARTICULIERS

Dans les exemples qui suivent, on utilise le 2,4-dinitrotrifluorométhoxybenzène (DNTFMB) comme composé nitré, en raison de sa grande similitude avec le dinitrotoluène (DNT) qui est le dérivé nitré le plus présent dans la signature chimique des mines à base de trinitrotoluène (TNT).

Par ailleurs, dans les exemples 1 à 4 et 6, les mesures d'intensité de fluorescence sont réalisées au moyen d'un fluorimètre FluoroMax-3 de la société JOBIN YVON, en régime dynamique dans une cellule balayée à 20 L/h. Ces mesures sont effectuées en utilisant la longueur d'onde d'excitation du matériau sensible conduisant au meilleur rapport signal/bruit pour l'acquisition des intensités de fluorescence, et à la longueur d'onde d'émission donnant les intensités de fluorescence maximales pour cette longueur d'onde d'excitation. Les longueurs d'onde d'émission et d'excitation ainsi retenues sont précisées dans chaque exemple.

### Exemple 1 : détection du DNTFMB par un premier exemple de capteur

Dans cet exemple, on réalise un capteur dont le fonctionnement est basé sur la variation de l'intensité de la fluorescence émise par le matériau sensible que comporte ce capteur en présence d'un composé nitré.

En l'espèce, le matériau sensible est constitué par un polymère à motif répétitif de formule particulière (I-A-a) dans laquelle $R^5$ à $R^{13}$ et $R^{16}$ = H, $R^{14}$ et $R^{15}$ = $OC_8H_{17}$, sous forme d'un film mince qui recouvre l'une des faces d'un substrat en verre de qualité optique.

Pour ce faire, le polymère est synthétisé à partir du (*1R*,*2R*)-diaminocyclohexane et du 4-bromo-benzaldéhyde comme décrit par J.P. Lère-Porte et *al.* dans la référence **[5]**, puis déposé sur le substrat en verre en procédant à trois pulvérisations de 0,2 seconde chacune d'une solution dudit polymère dans le chloroforme, de concentration égale à 1,5 g/L.

Le film mince ainsi obtenu présente une intensité de fluorescence de $2.10^7$ cps (coups/seconde ; $\lambda_{\text{émission}}$ : 507 nm ; $\lambda_{\text{excitation}}$ : 404 nm).

Le capteur est exposé successivement à :

- de l'azote pur pendant 30 minutes,
- du DNTFMB à une concentration de 0,5 ppm dans de l'azote pendant 10 minutes,
- de l'azote pur pendant 60 minutes,
- du DNTFMB à une concentration de 0,1 ppm dans de l'azote pendant 10 minutes,
- de l'azote pur pendant 50 minutes,
- du DNTFMB à une concentration de 0,03 ppm dans de l'azote pendant 10 minutes, et enfin à
- de l'azote pur pendant 15 minutes, l'azote et le DNTFMB étant, dans tous les cas, sous forme gazeuse et à température ambiante.

La figure 1 illustre l'évolution de l'intensité de fluorescence émise par le capteur au cours de ces expositions ($\lambda_{\text{émission}}$ : 507 nm ; $\lambda_{\text{excitation}}$ : 404 nm).

Sur cette figure, la courbe A représente les valeurs de l'intensité de fluorescence (I), exprimées en cps, en fonction du temps (t), exprimé en secondes, tandis que la courbe B représente les valeurs de la concentration en DNTFMB (C), exprimées en ppm, également en fonction du temps.

**Exemple 2 : mise en évidence de la sélectivité du premier exemple de capteur pour des composés nitrés vis-à-vis des solvants**

Dans cet exemple, on utilise un capteur identique à celui décrit dans l'exemple 1.

On expose ce capteur successivement à :

- de l'azote pur pendant 90 minutes,
- du DNTFMB à une concentration de 1 ppm dans de l'azote pendant 10 minutes,
- de l'azote pur pendant 30 minutes,
- du dichlorométhane à une concentration de 675 ppm dans de l'azote pendant 10 minutes,
- de l'azote pur pendant 30 minutes,
- du cyclohexane à une concentration de 540 ppm dans de l'azote pendant 10 minutes,
- de l'azote pur pendant 30 minutes,
- de la méthyléthylcétone à une concentration de 360 ppm dans de l'azote pendant 10 minutes,
- de l'azote pur pendant 80 minutes,
- du toluène à une concentration de 180 ppm dans de l'azote pendant 10 minutes,
- de l'azote pur pendant 25 minutes,
- de la méthylisobutylcétone à une concentration de 90 ppm dans de l'azote pendant 10 minutes,
- de l'azote pur pendant 30 minutes,
- de l'acétate d'éthyle à une concentration de 720 ppm dans de l'azote pendant 10 minutes,
- de l'azote pur pendant 25 minutes, et enfin à
- du DNTFMB à une concentration de 1 ppm dans de l'azote pendant 6 minutes,

l'azote, le DNTFMB et les autres solvants étant, dans tous les cas, sous forme gazeuse et à température ambiante.

La figure 2 illustre l'évolution de l'intensité de fluorescence (I), exprimée en cps ($\lambda_{\text{émission}}$ : 507 nm ; $\lambda_{\text{excitation}}$ : 404 nm), telle qu'émise par le capteur en fonction du temps (t), exprimé en secondes. La flèche f1 signale le début de la première exposition au mélange azote/DNTFMB ; la flèche f2 signale le début de l'exposition au mélange azote/dichlorométhane ; la flèche f3 signale la fin de l'exposition au mélange azote/acétate d'éthyle, tandis que la flèche f4 signale le début de la deuxième exposition au mélange azote/ DNTFMB.

Cette figure montre que l'exposition du capteur à des solvants tels que le dichlorométhane, le cyclohexane, la méthyléthylcétone ou le toluène, ne provoque pas de réponse du capteur comparable à celle obtenue lorsque ce dernier est exposé à un composé nitré. De plus, la réponse du capteur obtenue lors de sa deuxième exposition au DNTFMB

indique que les solvants n'ont pas affecté les performances du capteur vis-à-vis des composés nitrés.

**Exemple 3 : mise en évidence du maintien dans le temps des propriétés de détection du premier exemple de capteur**

Dans cet exemple, on utilise un capteur identique à celui décrit dans l'exemple 1.

Ce capteur est soumis à une série d'expositions à du DNTFMB à une concentration de 1 ppm dans de l'azote de 10 minutes chacune, la première exposition ayant lieu le jour du dépôt du film mince de polymère sur le substrat en verre (J0), et les suivantes à intervalles de temps sur une période de 96 jours. Entre deux expositions au DNTFMB, le capteur est conservé à l'air ambiant.

La figure 3 illustre les valeurs des variations de l'intensité de fluorescence ($\Delta I$) émise par le capteur lors des expositions au mélange air ambiant/DNTFMB effectuées à J0, J8, J42 et à J96 ($\lambda_{émission}$ : 507 nm ; $\lambda_{excitation}$ : 404 nm), ces valeurs étant déterminées pour chaque exposition comme suit :

$$\Delta I = \text{intensité de fluorescence émise au temps } t_0 \text{ d'une exposition} - \text{intensité de fluorescence émise au temps } t_{10min} \text{ de cette même exposition.}$$

Cette figure montre que, bien que la variation de l'intensité de fluorescence émise par le capteur tende à chuter au cours du temps, ce dernier est toujours apte à détecter du DNTFMB à la concentration de 1 ppm, 96 jours après le dépôt du film mince du polymère.

**Exemple 4 : détection du DNTFMB par un deuxième exemple de capteur**

Dans cet exemple, on réalise un capteur, dont le fonctionnement est également basé sur la variation de l'intensité de la fluorescence émise par le matériau sensible de ce capteur en présence d'un composé nitré et dans lequel le matériau sensible est constitué par un polymère à motif répétitif de formule (I-A-b) dans laquelle $R^5$ à $R^{13}$ et $R^{16}$ = H, $R^{14}$ et $R^{15}$ = $OC_8H_{17}$, sous forme d'un film mince qui recouvre l'une des faces d'un substrat en verre de qualité optique.

Le polymère est synthétisé à partir du (*1R,2R*)-diaminocyclohexane et du 4-bromobenzaldéhyde comme décrit par J.P. Lère-Porte et *al*. dans la référence [6], puis déposé sur le substrat en verre par dépôt à la goutte d'une solution dudit polymère dans le chlorure de méthylène, de concentration égale à 1 g/L.

L'évaporation du solvant est réalisée à température ambiante sous pression atmosphérique de façon à obtenir un film mince présentant une intensité de fluorescence de $3{,}5.10^6$ cps ($\lambda_{émission}$ = 500 nm ; $\lambda_{excitation}$ = 397 nm).

Le capteur est exposé successivement à :

* de l'azote pur pendant 45 minutes,
* du DNTFMB à une concentration de 1 ppm dans de l'azote pendant 10 minutes,
* de l'azote pur pendant 60 minutes,
* du DNTFMB à une concentration de 0,1 ppm dans de l'azote pendant 10 minutes, et enfin à
* de l'azote pur pendant 40 minutes, l'azote et le DNTFMB étant, dans tous les cas, sous forme gazeuse et à température ambiante.

La figure 4 illustre l'évolution de l'intensité de fluorescence émise par le capteur au cours de ces expositions ($\lambda_{émission}$ : 500 nm ; $\lambda_{excitation}$ : 397 nm).

Sur cette figure, la courbe A représente les valeurs de l'intensité de fluorescence (I), exprimées en cps, en fonction du temps (t), exprimé en secondes, tandis que la courbe B représente les valeurs de la variation de la concentration en DNTFMB (C), exprimées en ppm, également en fonction du temps.

**Exemple 5 : détection du DNTFMB par un troisième exemple de capteur**

Dans cet exemple, on réalise un capteur à microbalance à quartz.

Pour ce faire, on recouvre les deux faces d'un quartz de coupe AT de fréquence de vibration de 9 MHz muni de deux électrodes de mesure circulaires en or (modèle QA9RA-50, AMETEK PRECISION INSTRUMENTS) d'un film mince d'un polymère à motif répétitif de formule particulière (I-A-a) dans laquelle $R^5$ à $R^{13}$ et $R^{16}$ = H, $R^{14}$ et $R^{15}$ = $OC_8H_{17}$.

Ce film mince est obtenu en procédant sur chaque face du quartz à cinq pulvérisations de 0,2 seconde chacune d'une solution dudit polymère dans le chloroforme, de concentration égale à 1,5 g/L. La formation de ce film se traduit

par une variation de la fréquence de vibration du quartz de 0,6 kHz.

Le capteur est exposé successivement à :

* l'air ambiant pendant 9 minutes,
* du DNTFMB à une concentration de 3 ppm dans l'air ambiant, pendant 10 minutes,
* l'air ambiant pendant 38 minutes,
* du DNTFMB à une concentration de 3 ppm dans l'air ambiant, pendant 10 minutes, et enfin à
* l'air ambiant pendant 15 minutes.

La figure 5 illustre l'évolution de la fréquence de vibration du quartz au cours de ces expositions.

Sur cette figure, la courbe A représente les valeurs de la fréquence de vibration (F), exprimées en Hz (hertz), en fonction du temps (t), exprimé en secondes, tandis que la courbe B représente les valeurs de la concentration en DNTFMB (C), exprimées en ppm, également en fonction du temps.

**Exemple 6 : détection du DNTFMB par un quatrième exemple de capteur**

Dans cet exemple, on réalise un capteur, dont le fonctionnement est basé sur la variation de l'intensité de la fluorescence émise par le matériau sensible de ce capteur en présence d'un composé nitré et dans lequel le matériau sensible est constitué par un polymère à motif répétitif de formule (I-B-a) dans laquelle $R^5$, $R^6$, $R^9$, $R^{10}$, $R^{13}$ et $R^{16}$ = H, $R^{14}$ et $R^{15}$ = $OC_8H_{17}$, sous forme d'un film mince qui recouvre l'une des faces d'un substrat en verre de qualité optique.

Le polymère est synthétisé à partir du (*1R*,*2R*)-diaminocyclohexane et du 5-bromothiophène-2-carboxaldéhyde comme décrit par J.P. Lère-Porte et *al.* dans la référence **[5]**, puis déposé sur le substrat en verre en procédant à 4 pulvérisations de 0,15 seconde chacune d'une solution dudit polymère dans le tétrahydrofurane, de concentration égale à 3 g/L.

L'évaporation du solvant est réalisée à température ambiante sous pression atmosphérique de façon à obtenir un film mince présentant une intensité de fluorescence de $2.10^7$ cps ($\lambda_{émission}$ = 491 nm ; $\lambda_{excitation}$ = 397 nm).

Le capteur est exposé successivement à :

* de l'azote pur pendant 25 minutes,
* du DNTFMB à une concentration de 400 ppb dans de l'azote pendant 5 minutes, et à
* de l'azote pur pendant 100 minutes, l'azote et le DNTFMB étant sous forme gazeuse et à température ambiante.

La figure 6 illustre l'évolution de l'intensité de fluorescence émise par le capteur au cours de ces expositions ($\lambda_{émission}$ : 491 nm ; $\lambda_{excitation}$ : 397 nm).

Sur cette figure, la courbe A représente les valeurs de l'intensité de fluorescence (I), exprimées en cps, en fonction du temps (t), exprimé en secondes, tandis que la courbe B représente les valeurs de la variation de la concentration en DNTFMB (C), exprimées en ppm, également en fonction du temps.

**REFERENCES CITEES**

[1] M.J. Sailor et al., SPIE Proceeding, The International Society of Optical Engineering, 3713, 1999, 54-65
[2] K.J. Albert et D.R. Walt, Anal. Chem., 72, 2000, 1947
[3] B. Valeur, Molecular Fluorescence : Principles and Applications, 2002, Ed. WILEY VCH, New York
[4] J.A.O. Sanchez-Pedrono et al., Anal. Chem. Acta, 182, 1986, 285
[5] J.P. Lère-Porte et al., Chem. Commun., 24, 2002, 3020-3021
[6] J.P. Lère-Porte et al., Tet. Lett., 42, 2001, 3073-3076

**Revendications**

1. Utilisation d'un capteur chimique comprenant, en tant que matériau sensible, au moins un polymère comprenant au moins un motif répétitif répondant à la formule générale (I) ci-après :

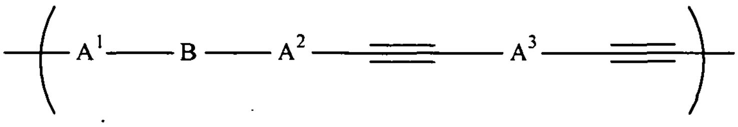

(I)

dans laquelle :

$A^1$, $A^2$ et $A^3$ représentent, indépendamment les uns des autres, un groupe phényle ou thiényle substitué ou non substitué ; et
B représente un groupe chiral de symétrie C2, substitué ou non substitué, ou bien un groupe répondant à l'une des formules (a), (b) et (c) ci-après :

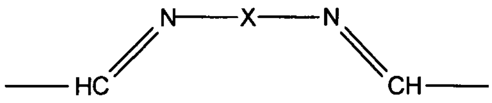

(a)

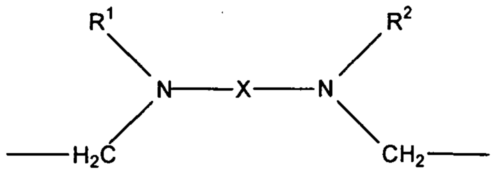

(b)

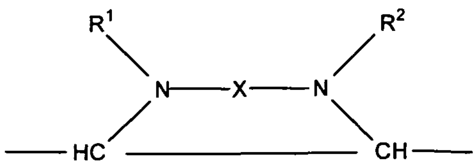

(c)

où :

X représente un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ;
$R^1$ et $R^2$ représentent indépendamment l'un de l'autre :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore
- une fonction chimique comprenant au moins un hétéroatome ;

pour la détection ou le dosage d'un ou plusieurs composés nitrés.

2. Utilisation selon la revendication 1, dans laquelle le polymère comprend au moins un motif répétitif de formule générale (I) dans laquelle $A^1$, $A^2$ et $A^3$ sont chacun un groupe phényle.

3. Utilisation selon la revendication 2, dans laquelle le polymère comprend au moins un motif répétitif répondant à la

formule particulière (I-A) ci-après :

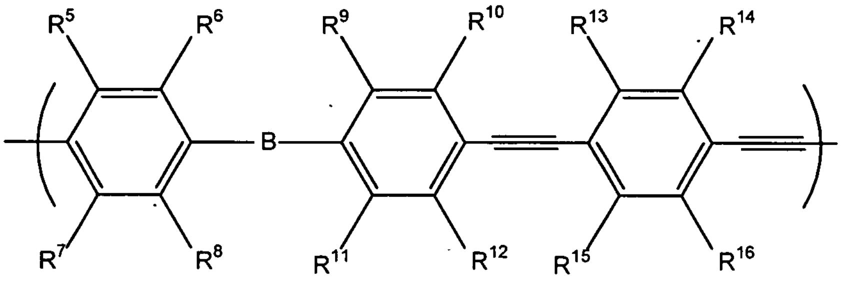

(I-A)

dans laquelle :

$R^5$, $R^6$, $R^7$, $R^8$, $R^9$, $R^{10}$, $R^{11}$, $R^{12}$, $R^{13}$, $R^{14}$, $R^{15}$ et $R^{16}$ représentent indépendamment les uns des autres :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore
- une fonction chimique comprenant au moins un hétéroatome ; et

B a la même signification que dans la revendication 1.

**4.** Utilisation selon la revendication 1, dans laquelle le polymère comprend au moins un motif répétitif de formule générale (I) dans laquelle $A^1$ et $A^2$ sont chacun un groupe thiényle et $A^3$ est un groupe phényle.

**5.** Utilisation selon la revendication 4, dans laquelle le polymère comprend au moins un motif répétitif répondant à la formule particulière (I-B) ci-après :

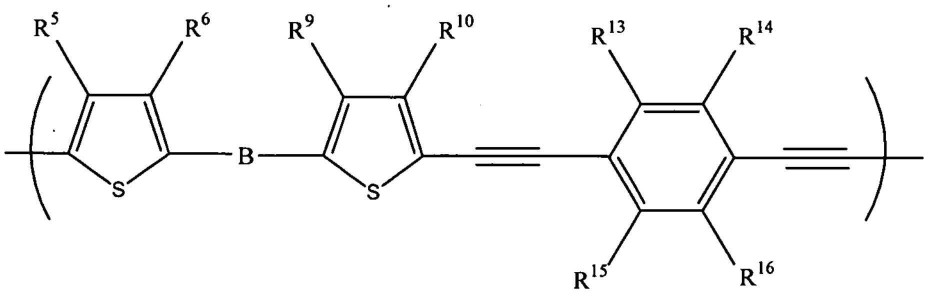

(I-B)

dans laquelle :

$R^5$, $R^6$, $R^9$, $R^{10}$, $R^{13}$, $R^{14}$, $R^{15}$ et $R^{16}$ représentent indépendamment les uns des autres :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique

ou hétéroaromatique, substitué ou non substitué, ou comprendre un ou plusieurs groupes aromatiques ou hétéroaromatiques, substitués ou non substitués ; ou encore

• une fonction chimique comprenant au moins un hétéroatome ; et

B a la même signification que dans la revendication 1.

**6.** Utilisation selon la revendication 1, dans laquelle le polymère comprend au moins un motif répétitif de formule générale (I) dans laquelle B représente un groupe répondant à l'une des formules (a), (b) et (c).

**7.** Utilisation selon la revendication 6, dans laquelle le polymère comprend au moins un motif répétitif de formule générale (I) dans laquelle B représente un groupe répondant à l'une des formules (a), (b) et (c) où X est un groupe hydrocarboné chiral de symétrie C2.

**8.** Utilisation selon la revendication 7, dans laquelle le polymère comprend un motif répétitif de formule générale (I) dans laquelle B représente un groupe répondant à l'une des formules (a), (b) et (c) où X est un groupe hydrocarboné choisi parmi les groupes de formules (i) à (vii) ci-après :

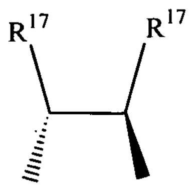

(i)

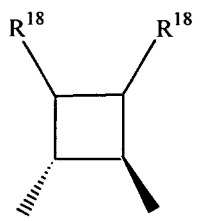

(ii)

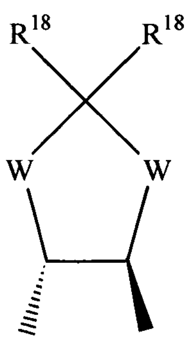

(iii)

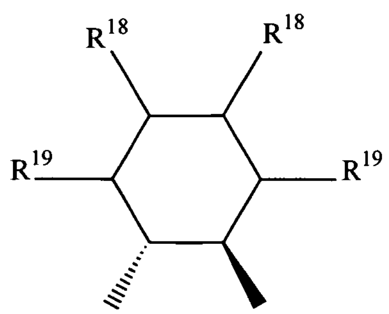

(iv)

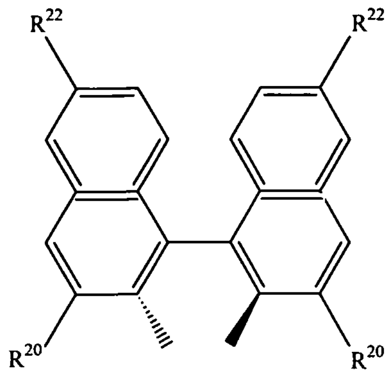

(v)

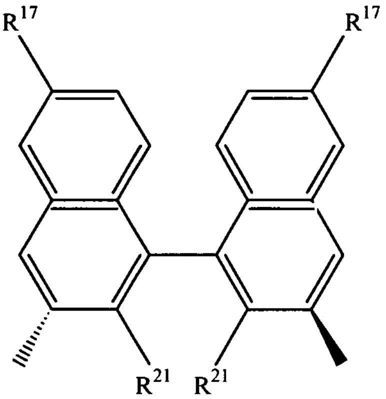

(vi)

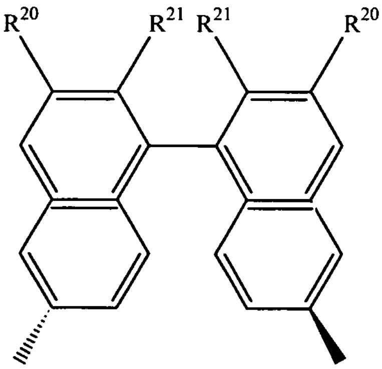

(vii)

dans lesquelles :

- $R^{17}$ représente un atome d'halogène ; un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome ;
- $R^{18}$, $R^{19}$, $R^{20}$ et $R^{21}$ représentent, indépendamment les uns des autres, un atome d'hydrogène ou d'halogène, un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome ;
- W représente un atome d'oxygène, un groupe -NH ou un groupe $-CH_2$, éventuellement substitué par un groupe hydrocarboné linaire, ramifié ou cyclique, saturé ou insaturé, comprenant de 1 à 100 atomes de carbone, ce

groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore une fonction chimique comprenant au moins un hétéroatome.

9. Utilisation selon la revendication 3, dans laquelle le polymère comprend au moins un motif répétitif répondant à l'une des formules particulières (I-A-a), (I-A-b) et (I-A-c) ci-après :

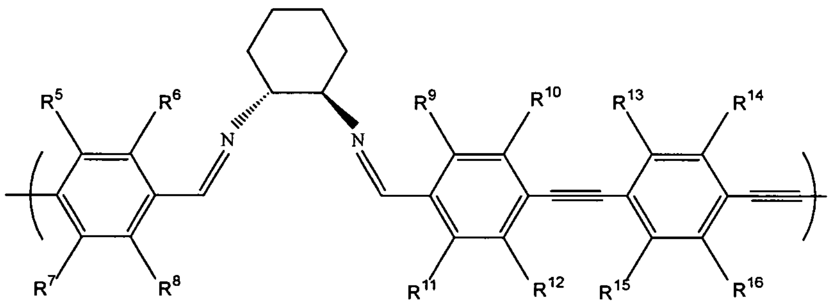

(I-A-a)

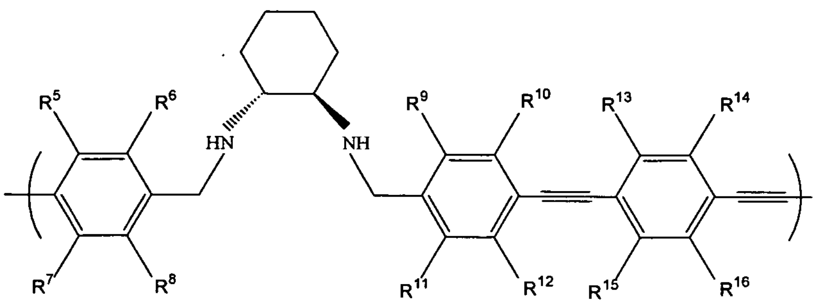

(I-A-b)

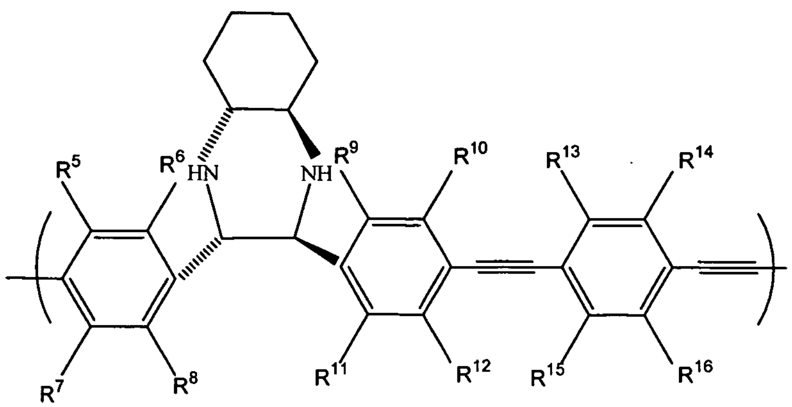

(I-A-c)

dans lesquelles $R^5$ à $R^{16}$ sont tels que définis dans la revendication 3.

10. Utilisation selon la revendication 3, dans laquelle le polymère comprend au moins un motif répétitif de formule

particulière (I-A) dans laquelle au moins l'un de $R^5$ à $R^{16}$ représente un groupe alcoxy, linéaire ou ramifié, en $C_1$ à $C_{20}$ et, mieux encore, en $C_5$ à $C_{10}$, celui ou ceux de $R^5$ à $R^{16}$ qui ne représentent pas un groupe alcoxy, s'il y en a, représentant alors un atome d'hydrogène.

**11.** Utilisation selon la revendication 9, dans laquelle le polymère comprend au moins un motif répétitif de formule particulière (I-A-a), (I-A-b) ou (I-A-c) dans laquelle $R^{14}$ et $R^{15}$ représentent un groupe alcoxy, linéaire ou ramifié, en $C_1$ à $C_{20}$ et, mieux encore, en $C_5$ à $C_{10}$, tandis que $R^5$ à $R^{13}$ et $R^{16}$ représentent un atome d'hydrogène.

**12.** Utilisation selon la revendication 11, dans laquelle le polymère comprend au moins un motif répétitif de formule particulière (I-A-a) ou (I-A-b) dans laquelle $R^{14}$ et $R^{15}$ représentent un groupe octoxy, tandis que $R^5$ à $R^{13}$ et $R^{16}$ représentent un atome d'hydrogène.

**13.** Utilisation selon la revendication 5, dans laquelle le polymère comprend au moins un motif répétitif répondant à l'une des formules particulières (I-B-a), (I-B-b) et (I-B-c) ci-après :

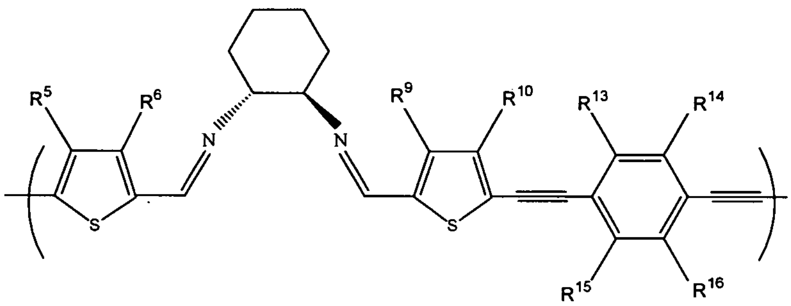

(I-B-a)

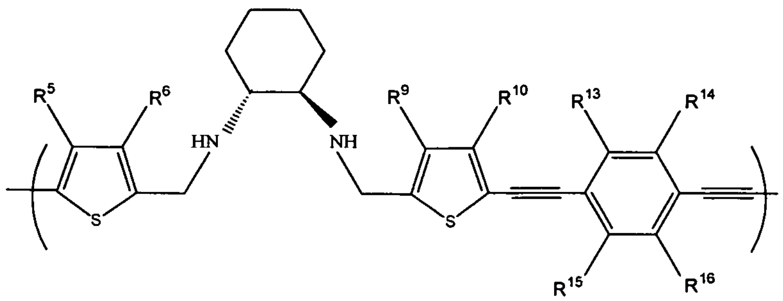

(I-B-b)

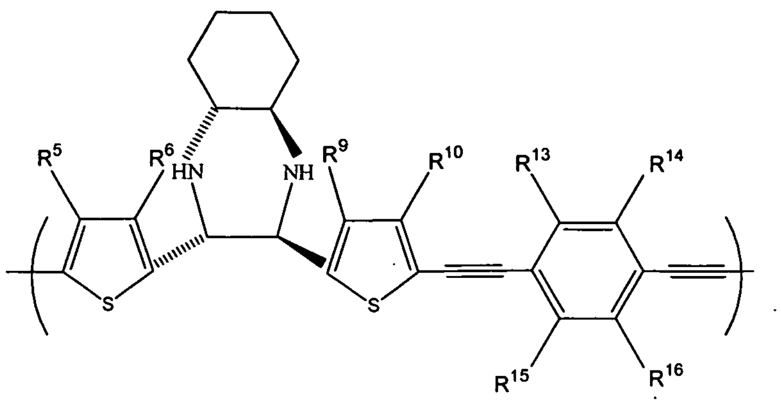

(I-B-c)

dans lesquelles $R^5$, $R^6$, $R^9$, $R^{10}$ et $R^{13}$ à $R^{16}$ sont tels que définis dans la revendication 5.

**14.** Utilisation selon la revendication 5, dans laquelle le polymère comprend au moins un motif répétitif de formule particulière (I-B) dans laquelle au moins l'un de $R^5$, $R^6$, $R^9$, $R^{10}$ et de $R^{13}$ à $R^{16}$ représente un groupe alcoxy, linéaire ou ramifié, en $C_1$ à $C_{20}$ et, mieux encore, en $C_5$ à $C_{10}$, celui ou ceux de $R^5$, $R^6$, $R^9$, $R^{10}$ et de $R^{13}$ à $R^{16}$ qui ne représentent pas un groupe alcoxy, s'il y en a, représentant alors un atome d'hydrogène.

**15.** Utilisation selon la revendication 13, dans laquelle le polymère comprend au moins un motif répétitif de formule particulière (I-B-a), (I-B-b) ou (I-B-c) dans laquelle $R^{14}$ et $R^{15}$ représentent un groupe alcoxy, linéaire ou ramifié, en $C_1$ à $C_{20}$ et, mieux encore, en $C_5$ à $C_{10}$, tandis que $R^5$, $R^6$, $R^9$, $R^{10}$, $R^{13}$ et $R^{16}$ représentent un atome d'hydrogène.

**16.** Utilisation selon la revendication 15, dans laquelle le polymère comprend au moins un motif répétitif de formule particulière (I-B-a) dans laquelle $R^{14}$ et $R^{15}$ représentent un groupe octoxy, tandis que $R^5$, $R^6$, $R^9$, $R^{10}$, $R^{13}$ et $R^{16}$ représentent un atome d'hydrogène.

**17.** Utilisation selon l'une quelconque des revendications précédentes, dans laquelle le polymère est un homopolymère.

**18.** Utilisation selon l'une quelconque des revendications précédentes, dans laquelle le polymère est présent sous la forme d'un film mince recouvrant l'une ou les deux faces d'un substrat, ce film mince mesurant de 10 angströms à 100 microns d'épaisseur.

**19.** Utilisation selon l'une quelconque des revendications précédentes, dans laquelle le capteur est un capteur optique à base de fluorescence ou un capteur gravimétrique.

**20.** Utilisation selon l'une quelconque des revendications précédentes, dans laquelle le ou les composés nitrés sont sous forme gazeuse.

**21.** Utilisation selon l'une quelconque des revendications précédentes, dans laquelle le ou les composés nitrés sont choisis parmi les composés nitroaromatiques, les nitramines, les nitrosamines et les esters nitriques.

**22.** Utilisation selon l'une quelconque des revendications précédentes pour la détection d'explosifs.

**23.** Capteur chimique comprenant, en tant que matériau sensible, au moins un polymère comprenant au moins un motif répétitif répondant à l'une des formules particulières (I-A-b) et (I-A-c) ci-après :

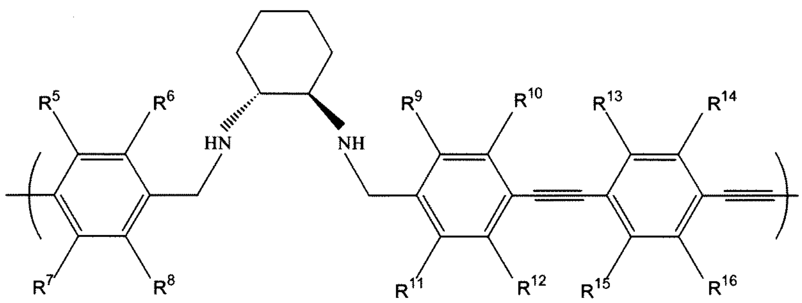

(I-A-b)

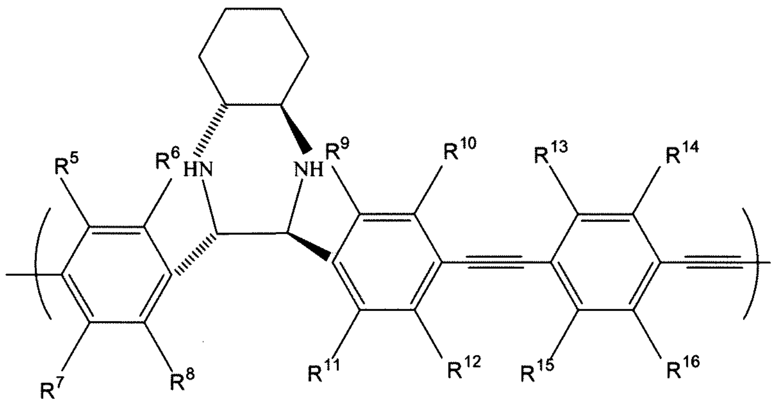

(I-A-c)

dans lesquelles $R^5$ à $R^{16}$ représentent indépendamment les uns des autres :

- un atome d'hydrogène ou d'halogène ;
- un groupe hydrocarboné linéaire, ramifié ou cyclique, saturé ou insaturé, comprenant de 1 à 100 atomes de carbone, ce groupe hydrocarboné pouvant comporter un ou plusieurs hétéroatomes et/ou une ou plusieurs fonctions chimiques comprenant au moins un hétéroatome, et pouvant être un groupe aromatique ou hétéroaromatique, substitué ou non substitué, ou comprendre un ou plusieurs groupes aromatiques ou hétéroaromatiques, substitués ou non substitués ; ou encore
- une fonction chimique comprenant au moins un hétéroatome.

**24.** Capteur selon la revendication 23, dans lequel le polymère comprend au moins un motif répétitif de formule particulière (I-A-b) ou (I-A-c) dans laquelle $R^{14}$ et $R^{15}$ représentent un groupe alcoxy, linéaire ou ramifié, en $C_1$ à $C_{20}$ et, mieux encore, en $C_5$ à $C_{10}$, tandis que $R^5$ à $R^{13}$ et $R^{16}$ représentent un atome d'hydrogène.

**25.** Capteur selon la revendication 23, dans lequel le polymère comprend au moins un motif répétitif de formule particulière (I-A-b) dans laquelle $R^{14}$ et $R^{15}$ représentent un groupe octoxy, tandis que $R^5$ à $R^{13}$ et $R^{16}$ représentent un atome d'hydrogène.

**26.** Capteur selon l'une quelconque des revendications 23 à 25, dans lequel le polymère est un homopolymère.

**27.** Capteur selon l'une quelconque des revendications 23 à 26, dans lequel le polymère est présent sous la forme d'un film mince recouvrant l'une ou les deux faces d'un substrat, ce film mince mesurant de 10 angströms à 100 microns d'épaisseur.

**28.** Capteur selon l'une quelconque des revendications 23 à 27, qui est un capteur optique à base de fluorescence ou un capteur gravimétrique.

**Patentansprüche**

**1.** Verwendung eines chemischen Sensors, der als empfindliches Material wenigstens ein Polymer umfasst, das wenigstens ein sich wiederholendes Motiv umfasst, das der nachfolgenden allgemeinen Formel (I) genügt:

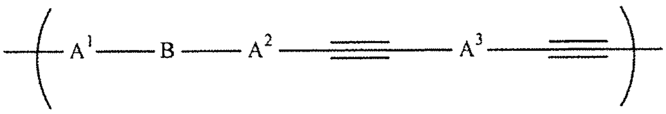

(I)

wobei:

$A^1$, $A^2$ und $A^3$ unabhängig voneinander eine Phenyl- oder Thienyl-Gruppe darstellen, die substituiert oder nicht substituiert ist; und
B eine Chiralgruppe der Symmetrie C2 darstellt, die substituiert oder nicht substituiert ist, oder aber eine Gruppe, die einer der nachfolgenden Formeln (a), (b) und (c) genügt:

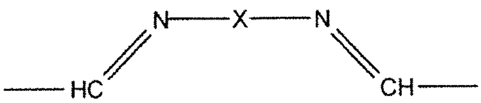

(a)

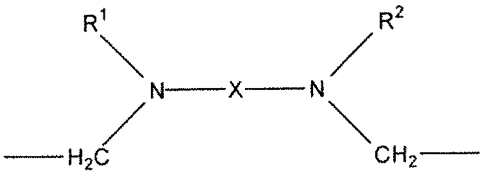

(b)

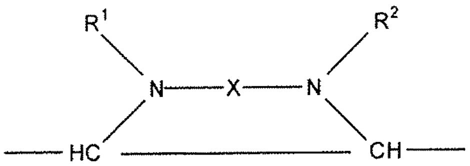

(c)

wobei:

X eine lineare, verzweigte oder zyklische Kohlenwasserstoffgruppe darstellt, gesättigt oder ungesättigt, die von 1 bis 100 Kohlenstoffatome umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert;
$R^1$ und $R^2$ unabhängig voneinander folgendes darstellen:

- ein Wasserstoff- oder Halogenatom
- eine lineare, verzweigte oder zyklische Kohlenwasserstoffgruppe, gesättigt oder ungesättigt, die von 1 bis 100 Kohlenstoffatome umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert; oder auch
- eine chemische Funktion, die wenigstens ein Heteroatom umfasst;

für die Erfassung oder die Dosierung einer oder mehrerer Nitroverbindungen.

2. Verwendung nach Anspruch 1, wobei das Polymer wenigstens ein sich wiederholendes Motiv der allgemeinen Formel (I) umfasst, wobei $A^1$, $A^2$ und $A^3$ jeweils eine Phenylgruppe sind.

3. Verwendung nach Anspruch 2, wobei das Polymer wenigstens ein sich wiederholendes Motiv umfasst, das der nachfolgenden speziellen Formel (I-A) genügt:

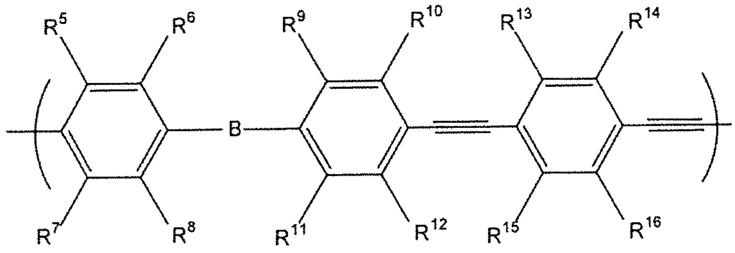

(I-A)

wobei:

$R^5$, $R^6$, $R^7$, $R^8$, $R^9$, $R^{10}$, $R^{11}$, $R^{12}$, $R^{13}$, $R^{14}$, $R^{15}$ und $R^{16}$ unabhängig voneinander folgendes darstellen

- ein Wasserstoff- oder Halogenatom
- eine lineare, verzweigte oder zyklische Kohlenwasserstoffgruppe, gesättigt oder ungesättigt, die von 1 bis 100 Kohlenstoffatome umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert; oder auch
- eine chemische Funktion, die wenigstens ein Heteroatom umfasst; und

B die gleiche Bedeutung wie in Anspruch 1 hat.

4. Verwendung nach Anspruch 1, wobei das Polymer wenigstens ein sich wiederholendes Motiv der allgemeinen Formel (I) umfasst, wobei $A^1$ und $A^2$ jeweils eine Thienylgruppe sind und $A^3$ eine Phenylgruppe ist.

5. Verwendung nach Anspruch 4, wobei das Polymer wenigstens ein sich wiederholendes Motiv umfasst, das der nachfolgenden speziellen Formel (I-B) genügt:

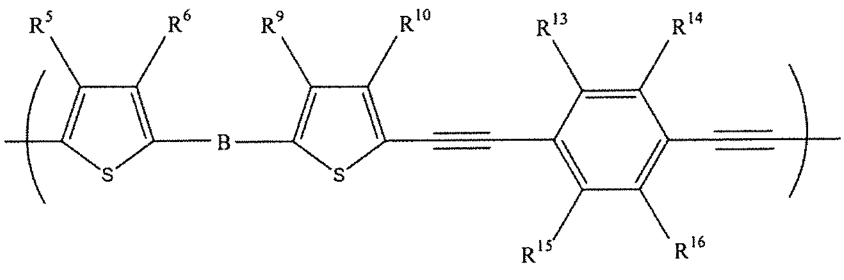

(I-B)

wobei:

$R^5$, $R^6$, $R^9$, $R^{10}$, $R^{13}$, $R^{14}$, $R^{15}$ und $R^{16}$ unabhängig voneinander folgendes darstellen:

- ein Wasserstoff- oder Halogenatom

• eine lineare, verzweigte oder zyklische Kohlenwasserstoffgruppe, gesättigt oder ungesättigt, die von 1 bis 100 Kohlenstoffatome umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert; oder auch

• eine chemische Funktion, die wenigstens ein Heteroatom umfasst; und

B die gleiche Bedeutung wie in Anspruch 1 hat.

**6.** Verwendung nach Anspruch 1, wobei das Polymer wenigstens ein sich wiederholendes Motiv der allgemeinen Formel (I) umfasst, wobei B eine Gruppe darstellt, die einer der Formeln (a), (b) und (c) genügt.

**7.** Verwendung nach Anspruch 6, wobei das Polymer wenigstens ein sich wiederholendes Motiv der allgemeinen Formel (I) umfasst, wobei B eine Gruppe darstellt, die einer der Formeln (a), (b) und (c) genügt, wobei X eine Chiral-Kohlenwasserstoffgruppe der Symmetrie C2 ist.

**8.** Verwendung nach Anspruch 7, wobei das Polymer ein sich wiederholendes Motiv der allgemeinen Formel (I) umfasst, wobei B eine Gruppe darstellt, die einer der Formeln (a), (b) und (c) genügt, wobei X eine Kohlenwasserstoffgruppe ist, die aus den Gruppen der nachfolgenden Formeln (i) bis (vii) ausgewählt ist:

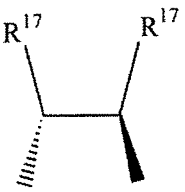

(i)

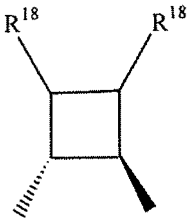

(ii)

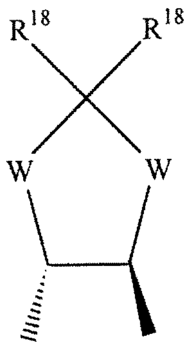

(iii)

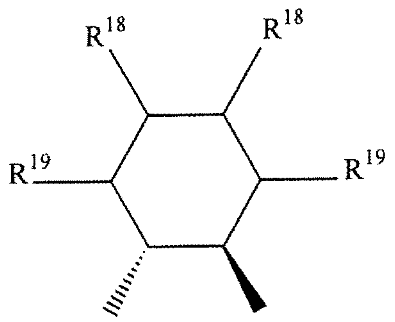

(iv)

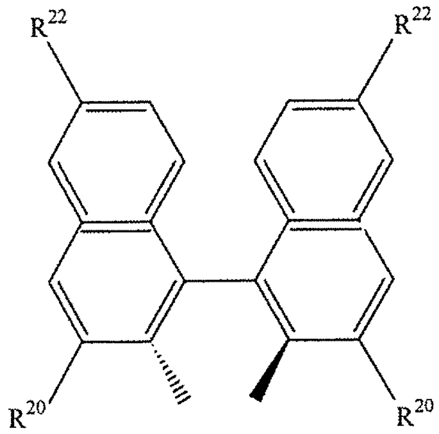

(v)

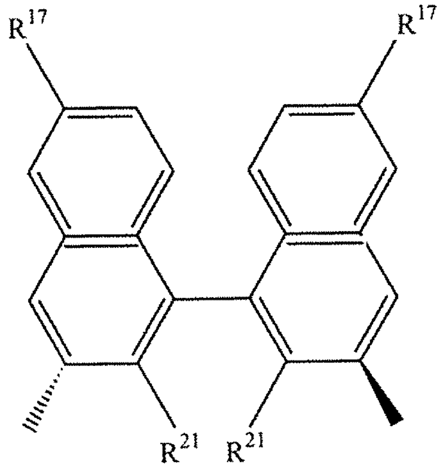

(vi)

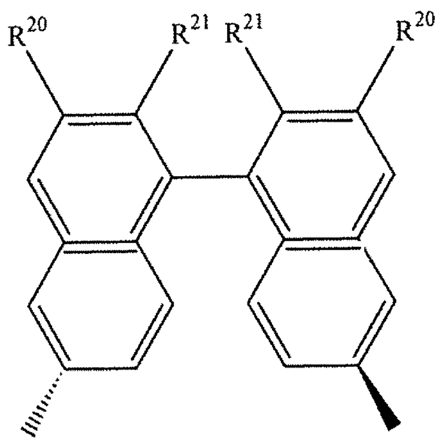

(vii)

wobei:

- $R^{17}$ ein Halogenatom darstellt, eine lineare, verzweigte oder zyklische Kohlenwasserstoffgruppe, gesättigt oder ungesättigt, umfassend von 1 bis 100 Kohlenstoffatome, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert, oder auch eine chemische Funktion, die wenigstens ein Heteroatom umfasst;
- $R^{18}$, $R^{19}$, $R^{20}$ und $R^{21}$ unabhängig voneinander ein Wasserstoff- oder Halogenatom darstellen, eine lineare, verzweigte oder zyklische Kohlenwasserstoffgruppe, gesättigt oder ungesättigt, die von 1 bis 100 Kohlenstoffatomen umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert, oder auch eine chemische Funktion, die wenigstens ein Heteroatom umfasst;
- W ein Sauerstoffatom darstellt, eine Gruppe -NH oder eine Gruppe-$CH_2$, gegebenenfalls substituiert durch eine lineare, verzweigte oder zyklische Kohlenwasserstoffgruppe, gesättigt oder ungesättigt, die von 1 bis 100 Kohlenstoffatomen umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert; oder auch eine chemische Funktion, die wenigstens ein Heteroatom umfasst.

**9.** Verwendung nach Anspruch 3, wobei das Polymer wenigstens ein sich wiederholendes Motiv umfasst, das einer der nachfolgenden speziellen Formeln (I-A-a), (I-A-b) und (I-A-c) genügt:

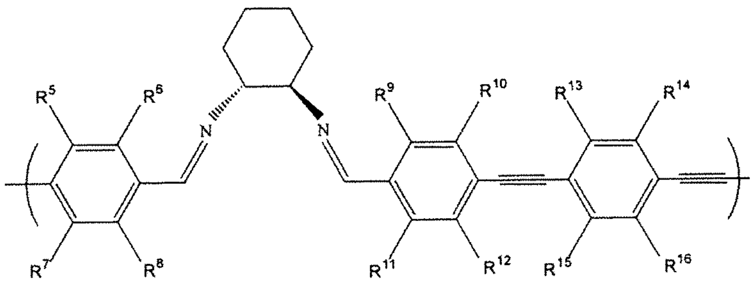

(I-A-a)

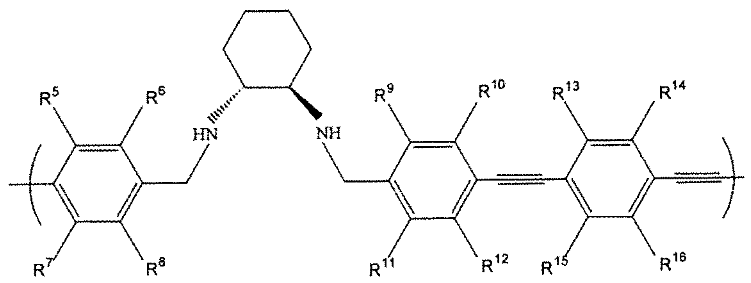

(I-A-b)

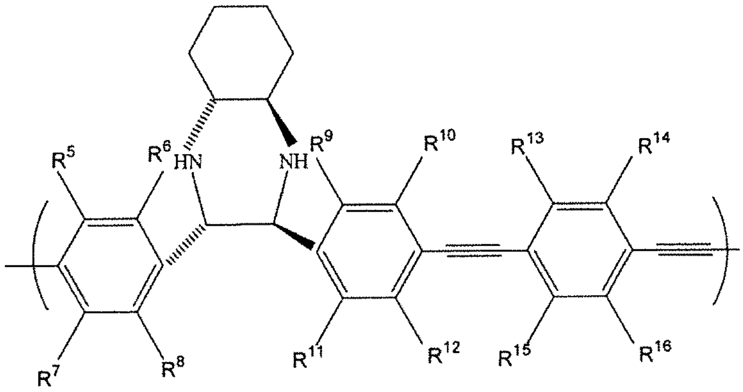

(I-A-c)

wobei $R^5$ bis $R^{16}$ wie in Anspruch 3 definiert sind.

**10.** Verwendung nach Anspruch 3, wobei das Polymer wenigstens ein sich wiederholendes Motiv der speziellen Formel (I-A) umfasst, wobei wenigstens eines der $R^5$ bis $R^{16}$ eine lineare oder verzweigte Alcoxy-Gruppe mit $C_1$ bis $C_{20}$ darstellt, und bevorzugt mit $C_5$ bis $C_{10}$, wobei diejenige oder diejenigen der $R^5$ bis $R^{16}$, die keine Alcoxy-Gruppe darstellen, falls vorhanden, dann ein Wasserstoffatom darstellen.

**11.** Verwendung nach Anspruch 9, wobei das Polymer wenigstens ein sich wiederholendes Motiv der speziellen Formel (I-A-a), (I-A-b) oder (I-A-c) umfasst, wobei $R^{14}$ und $R^{15}$ eine lineare oder verzweigte Alcoxy-Gruppe mit $C_1$ bis $C_{20}$ darstellen, und bevorzugt mit $C_5$ bis $C_{10}$, wohingegen $R^5$ bis $R^{13}$ und $R^{16}$ ein Wasserstoffatom darstellen.

**12.** Verwendung nach Anspruch 11, wobei das Polymer wenigstens ein sich wiederholendes Motiv der speziellen Formel (I-A-a) oder (I-A-b) umfasst, wobei $R^{14}$ und $R^{15}$ eine Octoxy-Gruppe darstellen, wohingegen $R^5$ bis $R^{13}$ und $R^{16}$ ein Wasserstoffatom darstellen.

**13.** Verwendung nach Anspruch 5, wobei das Polymer wenigstens ein sich wiederholendes Motiv umfasst, das einer nachfolgenden speziellen Formeln (I-B-a), (I-B-b) und (I-B-c) genügt:

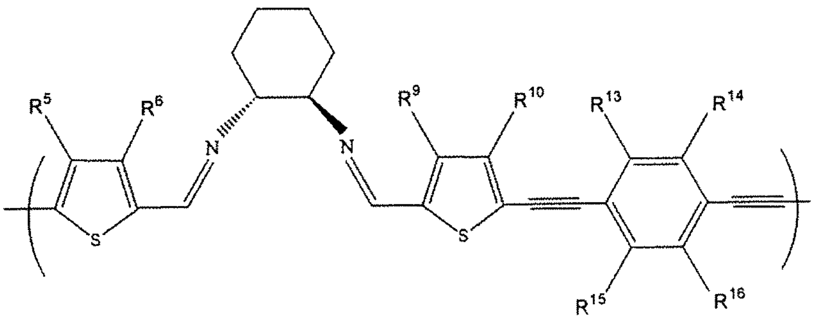

(I-B-a)

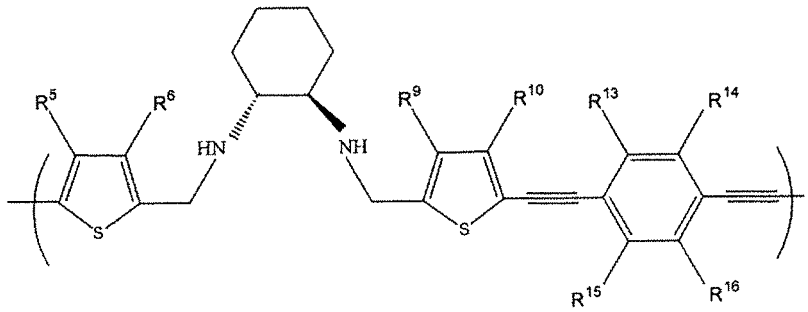

(I-B-b)

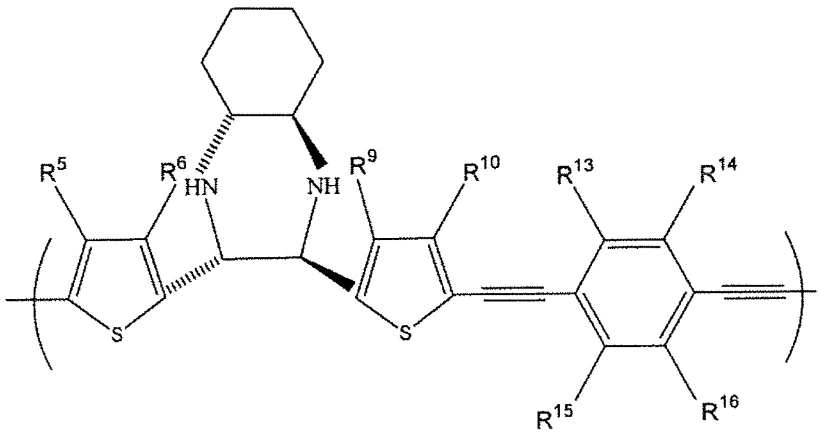

(I-B-c)

wobei $R^5$, $R^6$, $R^9$, $R^{10}$ und $R^{13}$ bis $R^{16}$ wie in Anspruch 5 definiert sind.

**14.** Verwendung nach Anspruch 5, wobei das Polymer wenigstens ein sich wiederholendes Motiv der speziellen Formel (I-B) umfasst, wobei wenigstens eines der $R^5$, $R^6$, $R^9$, $R^{10}$ und $R^{13}$ bis $R^{16}$ eine lineare oder verzweigte Alcoxy-Gruppe mit $C_1$ bis $C_{20}$ darstellen, und bevorzugt mit $C_5$ bis $C_{10}$, wohingegen dasjenige oder diejenigen der $R^5$, $R^6$, $R^9$, $R^{10}$ und $R^{13}$ bis $R^{16}$, die keine Alcoxy-Gruppe darstellen, falls vorhanden, dann ein Wasserstoffatom darstellen.

**15.** Verwendung nach Anspruch 13, wobei das Polymer wenigstens ein sich wiederholendes Motiv der speziellen Formel (I-B-a), (I-B-b) oder (I-B-c) umfasst, wobei $R^{14}$ und $R^{15}$ eine lineare oder verzweigte Alcoxy-Gruppe mit $C_1$ bis $C_{20}$ darstellen, und bevorzugt mit $C_5$ bis $C_{10}$, wohingegen $R^5$, $R^6$, $R^9$, $R^{10}$, $R^{13}$ und $R^{16}$ ein Wasserstoffatom darstellen.

**16.** Verwendung nach Anspruch 15, wobei das Polymer wenigstens ein sich wiederholendes Motiv der speziellen Formel (I-B-a) umfasst, wobei $R^{14}$ und $R^{15}$ eine Octoxy-Gruppe darstellen, wohingegen $R^5$, $R^6$, $R^9$, $R^{10}$, $R^{13}$ und $R^{16}$ ein Wasserstoffatom darstellen.

**17.** Verwendung nach einem der vorhergehenden Ansprüche, wobei das Polymer ein Homopolymer ist.

**18.** Verwendung nach einem der vorhergehenden Ansprüche, wobei das Polymer in Form eines dünnen Films vorhanden ist, der eine oder beide Flächen eines Substrats bedeckt, wobei dieser dünne Film in der Dicke von 10 Angström bis 100 Mikrometer misst.

**19.** Verwendung nach einem der vorhergehenden Ansprüche, wobei der Sensor ein optischer Sensor auf Fluoreszenzbasis oder ein gravimetrischer Sensor ist.

**20.** Verwendung nach einem der vorhergehenden Ansprüche, wobei die Nitroverbindung(en) gasförmig ist/sind.

**21.** Verwendung nach einem der vorhergehenden Ansprüche, wobei die Nitroverbindung(en) ausgewählt ist/sind aus den nitroaromatischen Verbindungen, den Nitraminen, den Nitrosaminen und den Nitratestern.

**22.** Verwendung nach einem der vorhergehenden Ansprüche zur Erfassung von Sprengstoffen.

**23.** Chemischer Sensor, der als empfindliches Material wenigstens ein Polymer umfasst, das wenigstens ein sich wiederholendes Motiv umfasst, das einer der nachfolgenden speziellen Formeln (I-A-b) und (I-A-c) genügt:

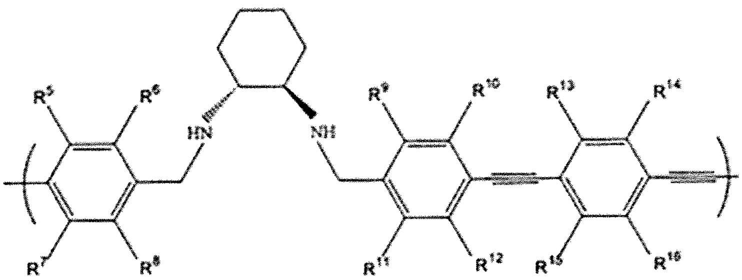

(I-A-b)

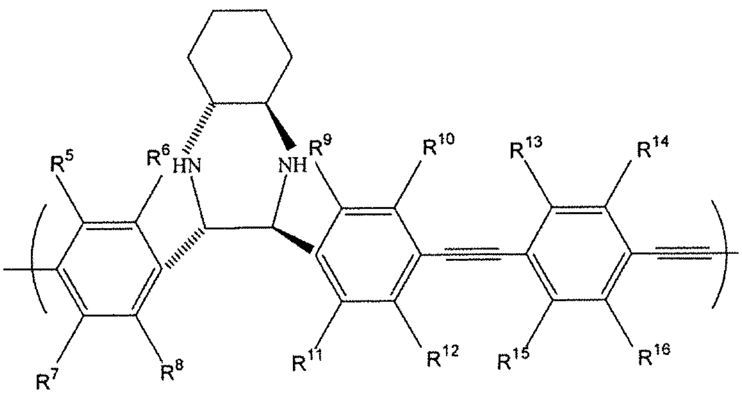

(I-A-c)

wobei $R^5$ bis $R^{16}$ unabhängig voneinander folgendes darstellen:

- ein Wasserstoff- oder Halogenatom
- eine lineare, verzweigte oder zyklische Kohlenwasserstoffgruppe, gesättigt oder ungesättigt, die von 1 bis 100 Kohlenstoffatome umfasst, wobei diese Kohlenwasserstoffgruppe ein oder mehrere Heteroatome und/oder eine oder mehrere chemische Funktionen umfassen kann, die wenigstens ein Heteroatom umfassen, und eine aromatische oder heteroaromatische Gruppe sein kann, substituiert oder nicht substituiert, oder eine oder mehrere aromatische oder heteroaromatische Gruppen umfassen kann, substituiert oder nicht substituiert; oder auch
- eine chemische Funktion, die wenigstens ein Heteroatom umfasst.

**24.** Sensor nach Anspruch 23, wobei das Polymer wenigstens ein sich wiederholendes Motiv der speziellen Formel (I-A-b) oder (I-A-c) umfasst, wobei $R^{14}$ und $R^{15}$ eine lineare oder verzweigte Alcoxy-Gruppe mit $C_1$ bis $C_{20}$ darstellen, und bevorzugt mit $C_5$ bis $C_{10}$, wohingegen $R^5$ bis $R^{13}$ und $R^{16}$ ein Wasserstoffatom darstellen.

**25.** Sensor nach Anspruch 23, wobei das Polymer wenigstens ein sich wiederholendes Motiv der speziellen Formel (I-A-b) umfasst, wobei $R^{14}$ und $R^{15}$ eine Octoxy-Gruppe darstellen, wohingegen $R^5$ bis $R^{13}$ und $R^{16}$ ein Wasserstoffatom darstellen.

**26.** Sensor nach einem der Ansprüche 23 bis 25, wobei das Polymer ein Homopolymer ist.

**27.** Sensor nach einem der Ansprüche 23 bis 26, wobei das Polymer in der Form eines dünnen Films vorhanden ist, der eine oder beide Flächen eines Substrats bedeckt, wobei dieser dünne Film in der Dicke von 10 Angström bis 100 Mikrometer misst.

**28.** Sensor nach einem der Ansprüche 23 bis 27, der ein optischer Sensor auf Fluoreszenzbasis oder ein gravimetrischer Sensor ist.

## Claims

**1.** Use of a chemical sensor comprising, as sensitive material, at least one polymer comprising at least one repeat unit corresponding to the general formula (I) below:

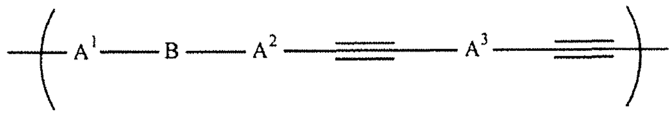

(I)

in which:

$A^1$, $A^2$ and $A^3$ represent, independently of one another, a substituted or unsubstituted phenyl or thienyl group; and B represents a substituted or unsubstituted chiral group of C2 symmetry or else a group corresponding to one of the formulae (a), (b) and (c) below:

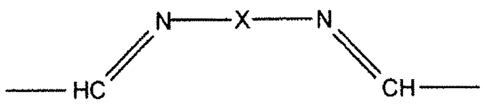

(a)

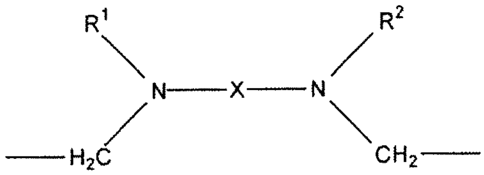

(b)

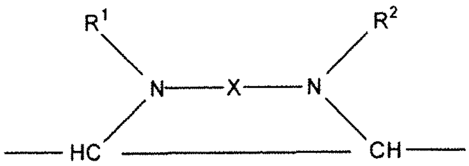

(c)

where:

X represents a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups;

$R^1$ and $R^2$ represent, independently of one another:

- a hydrogen or halogen atom;
- a saturated or unsaturated, linear, branched or cyclic, hydrocarbon group comprising from 1 to 100

carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or still
• a chemical functional group comprising at least one heteroatom;

in the detection or assaying of one or more nitro compounds.

**2.** Use according to Claim 1, in which the polymer comprises at least one repeat unit of general formula (I) in which $A^1$, $A^2$ and $A^3$ are each a phenyl group.

**3.** Use according to Claim 2, in which the polymer comprises at least one repeat unit corresponding to the specific formula (I-A) below:

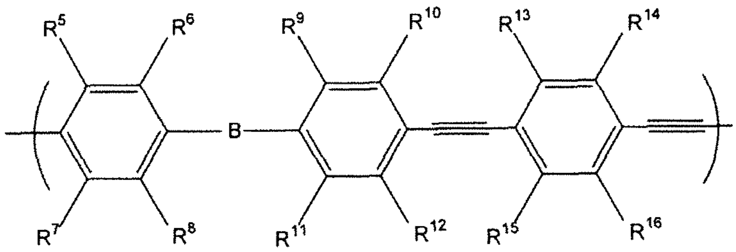

(I-A)

in which:

$R^5$, $R^6$, $R^7$, $R^8$, $R^9$, $R^{10}$, $R^{11}$, $R^{12}$, $R^{13}$, $R^{14}$, $R^{15}$ and $R^{16}$ represent, independently of one another:

• a hydrogen or halogen atom;
• a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or still
• a chemical functional group comprising at least one heteroatom; and

B has the same meaning as in Claim 1.

**4.** Use according to Claim 1, in which the polymer comprises at least one repeat unit of general formula (I) in which $A^1$ and $A^2$ are each a thienyl group and $A^3$ is a phenyl group.

**5.** Use according to Claim 4, in which the polymer comprises at least one repeat unit corresponding to the specific formula (I-B) below:

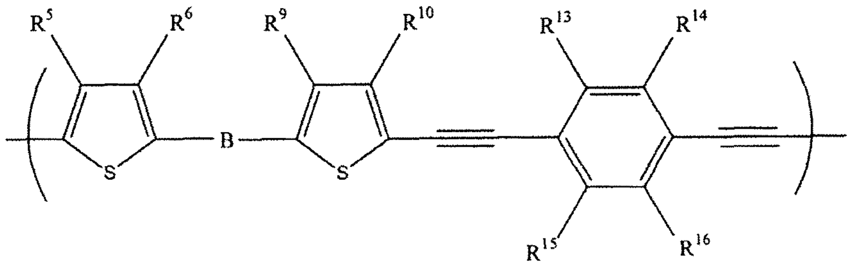

(I-B)

in which:

$R^5$, $R^6$, $R^9$, $R^{10}$, $R^{13}$, $R^{14}$, $R^{15}$ and $R^{16}$ represent, independently of one another:

- a hydrogen or halogen atom;
- a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or still
- a chemical functional group comprising at least one heteroatom; and

B has the same meaning as in Claim 1.

**6.** Use according to Claim 1, in which the polymer comprises at least one repeat unit of general formula (I) in which B represents a group corresponding to one of the formulae (a), (b) and (c).

**7.** Use according to Claim 6, in which the polymer comprises at least one repeat unit of general formula (I) in which B represents a group corresponding to one of the formulae (a), (b) and (c) where X is a chiral hydrocarbon group of C2 symmetry.

**8.** Use according to Claim 7, in which the polymer comprises a repeat unit of general formula (I) in which B represents a group corresponding to one of the formulae (a), (b) and (c) where X is a hydrocarbon group chosen from the groups of formulae (i) to (vii) below:

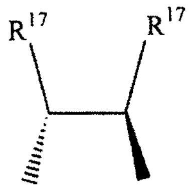

(i)

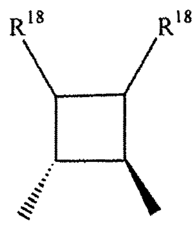

(ii)

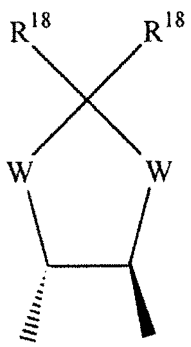

(iii)

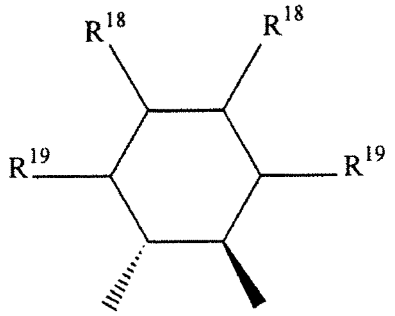

(iv)

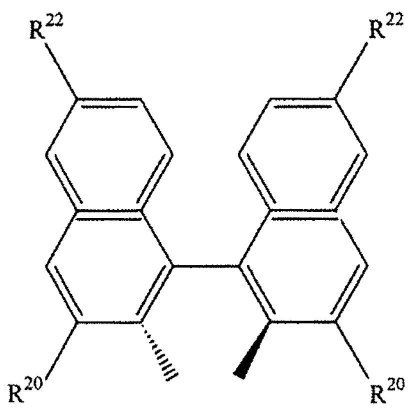

(v)

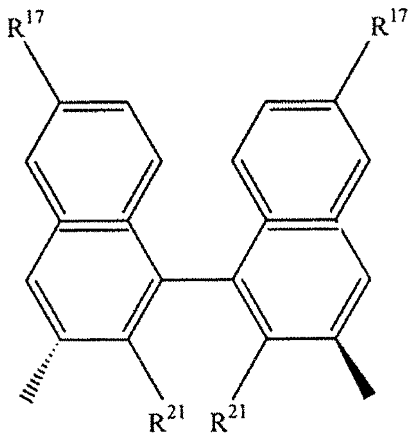

(vi)

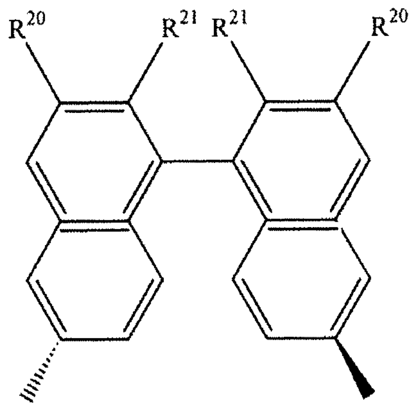

(vii)

in which:

- $R^{17}$ represents a halogen atom; a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or a chemical functional group comprising at least one heteroatom;
- $R^{18}$, $R^{19}$, $R^{20}$ and $R^{21}$ represent, independently of one another, a hydrogen or halogen atom; a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or a chemical functional group comprising at least one heteroatom;
- W represents an oxygen atom, an NH group or a $CH_2$ group which is optionally substituted by a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or a chemical functional group comprising at least one heteroatom.

**9.** Use according to Claim 3, in which the polymer comprises at least one repeat unit corresponding to one of the specific formulae (I-A-a), (I-A-b) and (I-A-c) below:

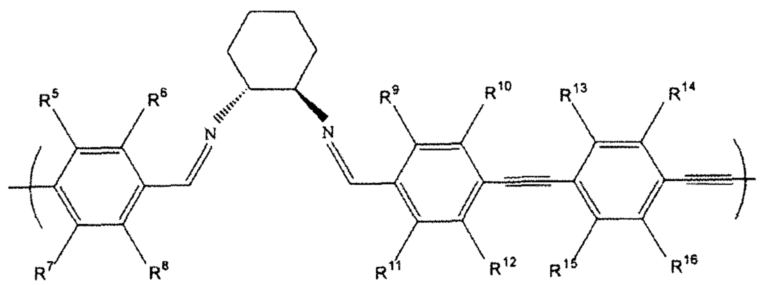

(I-A-a)

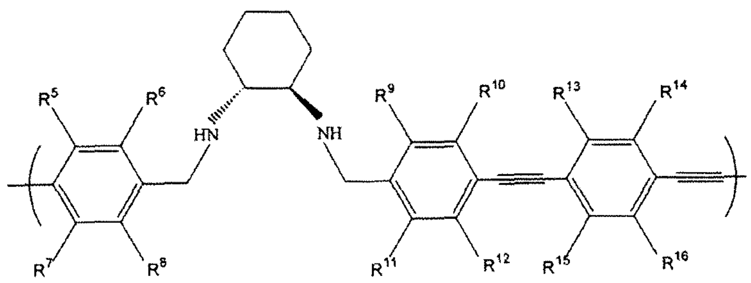

(I-A-b)

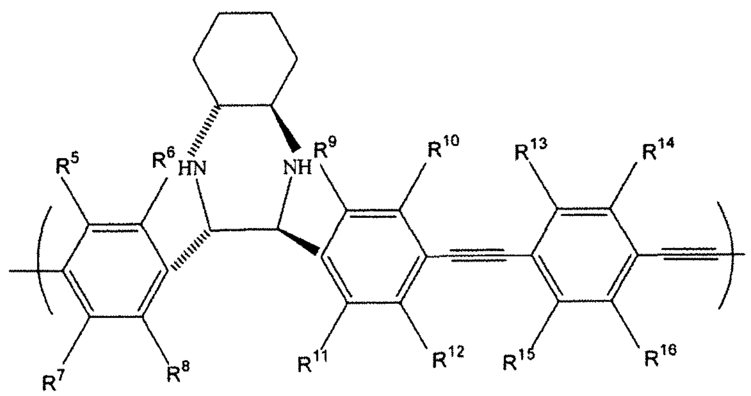

(I-A-c)

in which $R^5$ to $R^{16}$ are as defined in Claim 3.

**10.** Use according to Claim 3, in which the polymer comprises at least one repeat unit of specific formula (I-A) in which at least one of $R^5$ to $R^{16}$ represents a linear or branched $C_1$ to $C_{20}$ and better still $C_5$ to $C_{10}$ alkoxy group, that or those of $R^5$ to $R^{16}$ which do not represent an alkoxy group, if there are any, then representing a hydrogen atom.

**11.** Use according to Claim 9, in which the polymer comprises at least one repeat unit of specific formula (I-A-a), (I-A-b) or (I-A-c) in which $R^{14}$ and $R^{15}$ represent a linear or branched $C_1$ to $C_{20}$ and better still $C_5$ to $C_{10}$ alkoxy group while $R^5$ to $R^{13}$ and $R^{16}$ represent a hydrogen atom.

**12.** Use according to Claim 11, in which the polymer comprises at least one repeat unit of specific formula (I-A-a) or (I-A-b) in which $R^{14}$ and $R^{15}$ represent an octoxy group while $R^5$ to $R^{13}$ and $R^{16}$ represent a hydrogen atom.

**13.** Use according to Claim 5, in which the polymer comprises at least one repeat unit corresponding to one of the specific formulae (I-B-a), (I-B-b) and (I-B-c) below:

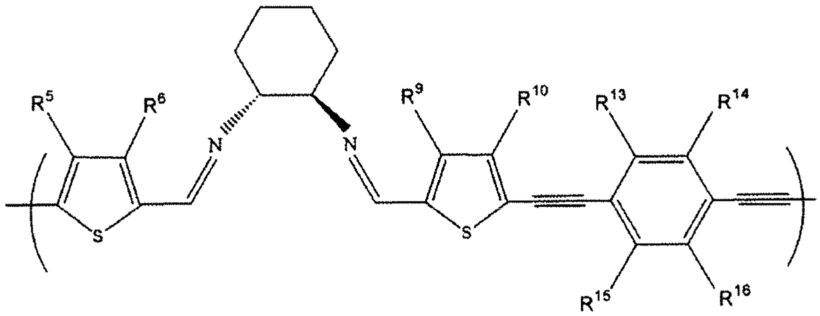

(I-B-a)

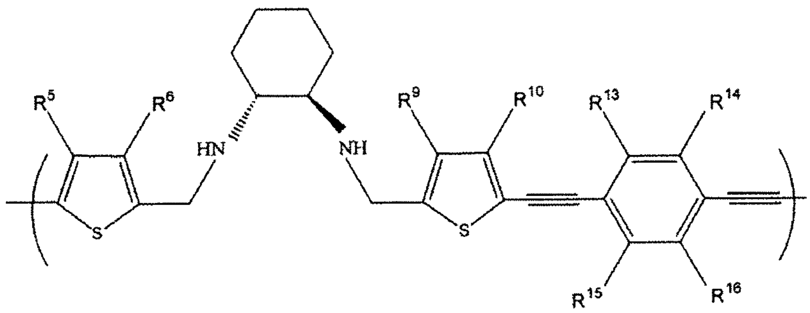

(I-B-b)

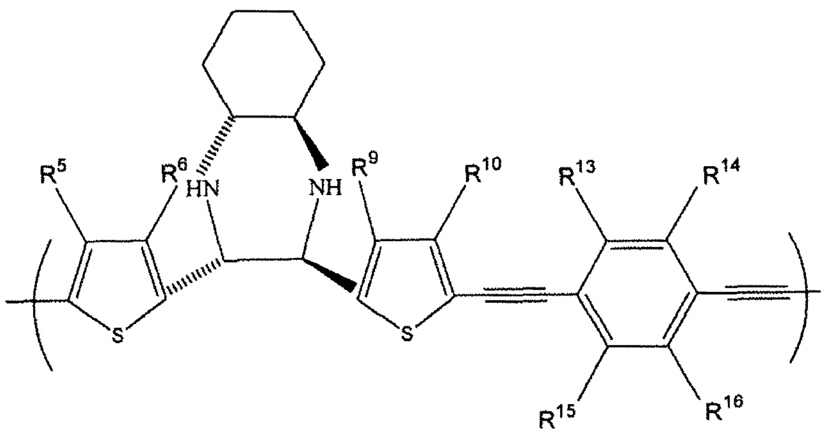

(I-B-c)

in which $R^5$, $R^6$, $R^9$, $R^{10}$ and $R^{13}$ to $R^{16}$ are as defined in Claim 5.

**14.** Use according to Claim 5, in which the polymer comprises at least one repeat unit of specific formula (I-B) in which at least one of $R^5$, $R^6$, $R^9$, $R^{10}$ and $R^{13}$ to $R^{16}$ represents a linear or branched $C_1$ to $C_{20}$ and better still $C_5$ to $C_{10}$ alkoxy group, that or those of $R^5$, $R^6$, $R^9$, $R^{10}$ and $R^{13}$ to $R^{16}$ which do not represent an alkoxy group, if there are any, then representing a hydrogen atom.

**15.** Use according to Claim 13, in which the polymer comprises at least one repeat unit of specific formula (I-B-a), (I-B-b) or (I-B-c) in which $R^{14}$ and $R^{15}$ represent a linear or branched $C_1$ to $C_{20}$ and better still $C_5$ to $C_{10}$ alkoxy group while $R^5$, $R^6$, $R^9$, $R^{10}$, $R^{13}$ and $R^{16}$ represent a hydrogen atom.

**16.** Use according to Claim 15, in which the polymer comprises at least one repeat unit of specific formula (I-B-a) in which $R^{14}$ and $R^{15}$ represent an octoxy group while $R^5$, $R^6$, $R^9$, $R^{10}$, $R^{13}$ and $R^{16}$ represent a hydrogen atom.

**17.** Use according to any one of the preceding claims, in which the polymer is a homopolymer.

**18.** Use according to any one of the preceding claims, in which the polymer is present in the form of a thin film covering one or both faces of a substrate, this thin film measuring from 10 angstroms to 100 microns in thickness.

**19.** Use according to any one of the preceding claims, in which the sensor is a fluorescence-based optical sensor or a gravimetric sensor.

**20.** Use according to any one of the preceding claims, in which the nitro compound or compounds are in the gas form.

**21.** Use according to any one of the preceding claims, in which the nitro compound or compounds are chosen from nitroaromatic compounds, nitramines, nitrosamines and nitric esters.

**22.** Use according to any one of the preceding claims for the detection of explosives.

**23.** Chemical sensor comprising, as sensitive material, at least one polymer comprising at least one repeat unit corresponding to the specific formulae (I-A-b) and (I-A-c) below:

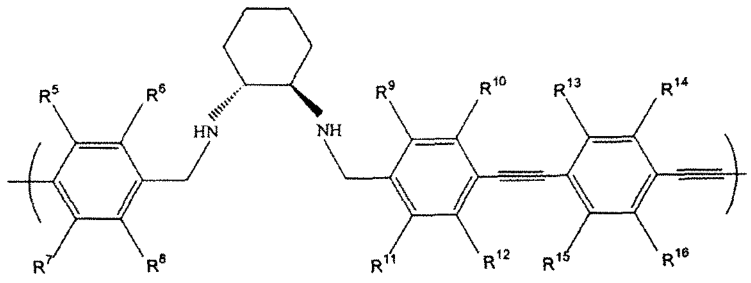

(I-A-b)

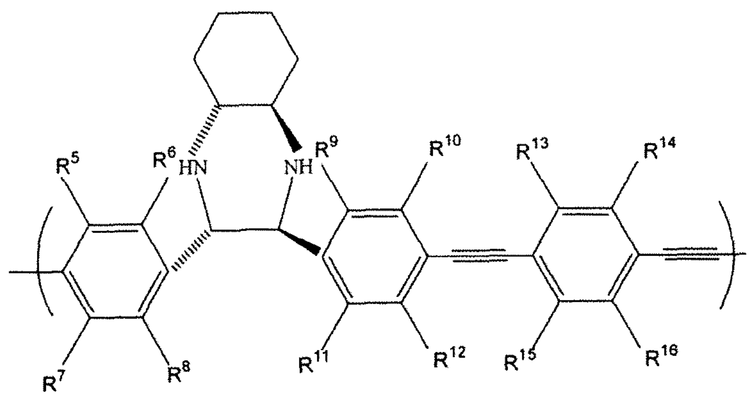

(I-A-c)

in which $R^5$ to $R^{16}$ represent, independently of one another:

- a hydrogen or halogen atom;
- a saturated or unsaturated, linear, branched or cyclic, hydrocarbon group comprising from 1 to 100 carbon atoms, which hydrocarbon group can comprise one or more heteroatoms and/or one or more chemical functional groups comprising at least one heteroatom, and can be a substituted or unsubstituted aromatic or heteroaromatic group or can comprise one or more substituted or unsubstituted aromatic or heteroaromatic groups; or

• a chemical functional group comprising at least one heteroatom.

**24.** Sensor according to Claim 23, in which the polymer comprises at least one repeat unit of specific formula (I-A-b) or (I-A-c) in which $R^{14}$ and $R^{15}$ represent a linear or branched $C_1$ to $C_{20}$ and better still $C_5$ to $C_{10}$ alkoxy group while $R^5$ to $R^{13}$ and $R^{16}$ represent a hydrogen atom.

**25.** Sensor according to Claim 23, in which the polymer comprises at least one repeat unit of specific formula (I-A-b) in which $R^{14}$ and $R^{15}$ represent an octoxy group while $R^5$ to $R^{13}$ and $R^{16}$ represent a hydrogen atom.

**26.** Sensor according to any one of claims 23 to 25, in which the polymer is a homopolymer.

**27.** Sensor according to any one of claims 23 to 26, in which the polymer is present in the form of a thin film covering one or both faces of a substrate, this thin film measuring from 10 angstroms to 100 microns in thickness.

**28.** Sensor according to any one of claims 23 to 27, which is a fluorescence-based optical sensor or a gravimetric sensor.

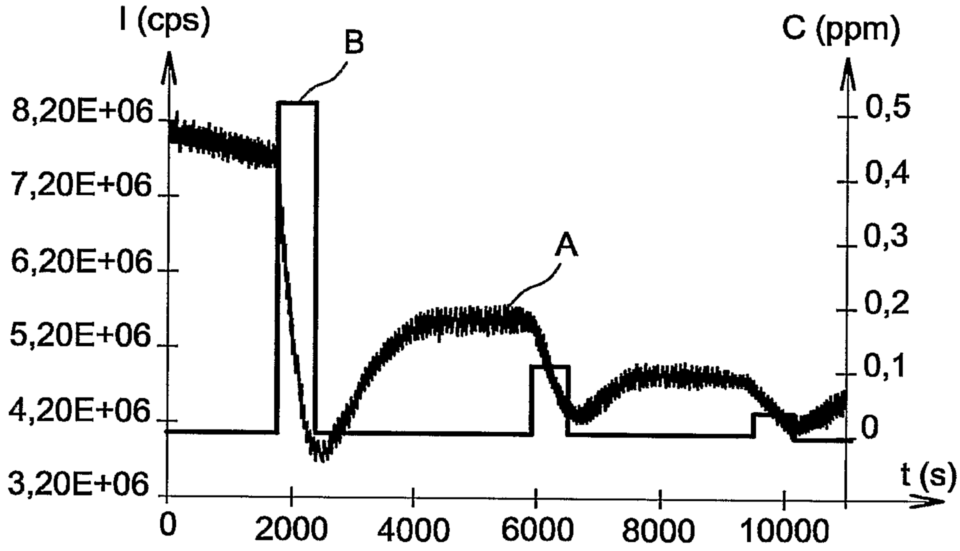
I (cps)
C (ppm)
B
A
8,20E+06
7,20E+06
6,20E+06
5,20E+06
4,20E+06
3,20E+06
0,5
0,4
0,3
0,2
0,1
0
0
2000
4000
6000
8000
10000
t (s)

FIG. 1

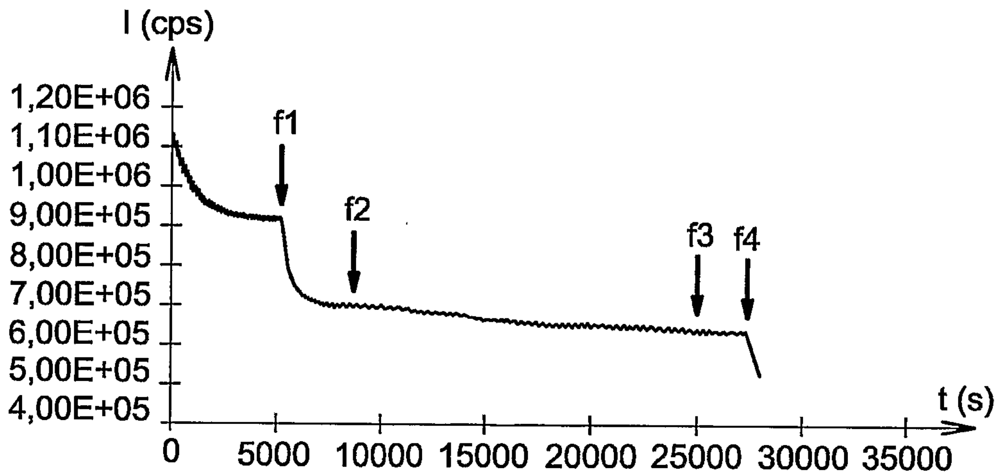
I (cps)
1,20E+06
1,10E+06
1,00E+06
9,00E+05
8,00E+05
7,00E+05
6,00E+05
5,00E+05
4,00E+05
f1
f2
f3
f4
0
5000
10000
15000
20000
25000
30000
35000
t (s)

FIG. 2

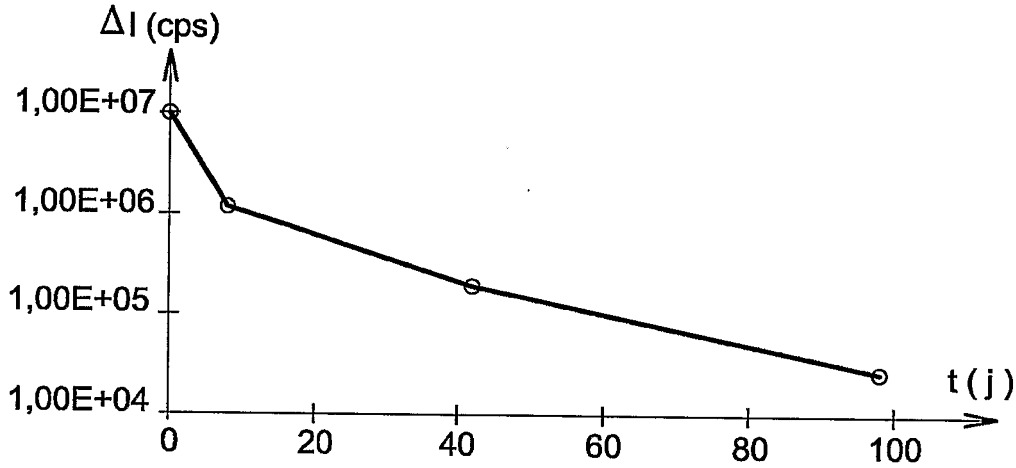
ΔI (cps)
1,00E+07
1,00E+06
1,00E+05
1,00E+04
0
20
40
60
80
100
t ( j )

FIG. 3

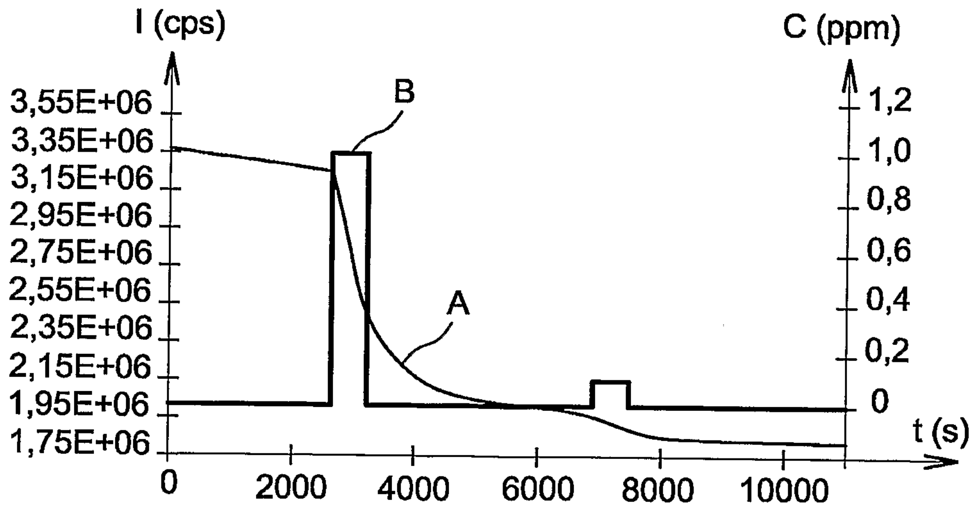
I (cps)
C (ppm)
3,55E+06
3,35E+06
3,15E+06
2,95E+06
2,75E+06
2,55E+06
2,35E+06
2,15E+06
1,95E+06
1,75E+06
B
A
1,2
1,0
0,8
0,6
0,4
0,2
0
t (s)
0
2000
4000
6000
8000
10000

FIG. 4

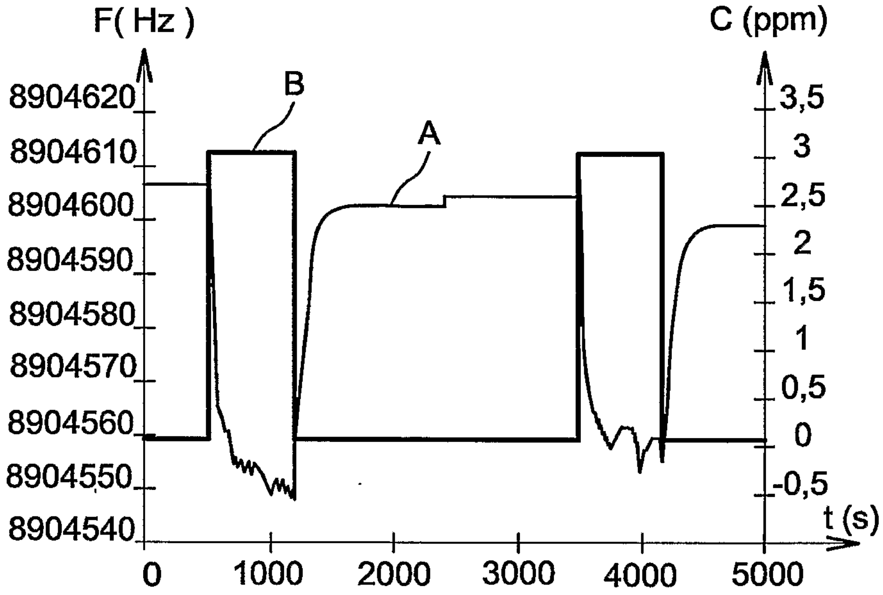
F( Hz )
C (ppm)
8904620
8904610
8904600
8904590
8904580
8904570
8904560
8904550
8904540
3,5
3
2,5
2
1,5
1
0,5
0
-0,5
B
A
t (s)
0
1000
2000
3000
4000
5000

FIG. 5

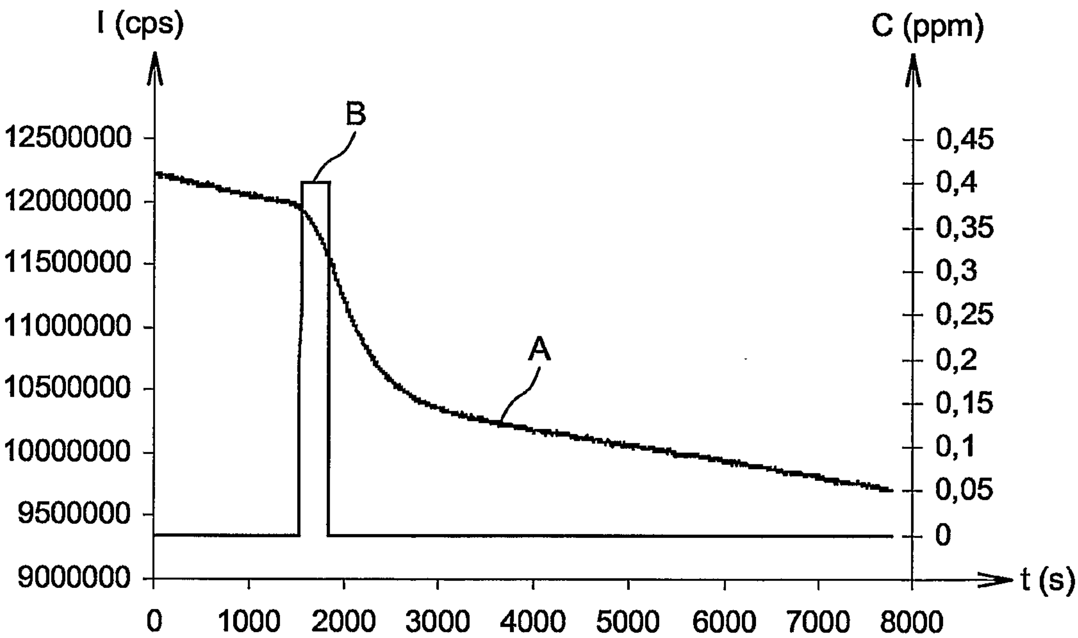
I (cps)
C (ppm)
12500000
12000000
11500000
11000000
10500000
10000000
9500000
9000000
0,45
0,4
0,35
0,3
0,25
0,2
0,15
0,1
0,05
0
B
A
t (s)
0
1000
2000
3000
4000
5000
6000
7000
8000

FIG. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **M.J. SAILOR et al.** SPIE Proceeding. The International Society of Optical Engineering, 1999, vol. 3713, 54-65
- **K.J. ALBERT ; D.R. WALT.** *Anal. Chem.,* 2000, vol. 72, 1947
- **B. VALEUR.** Molecular Fluorescence : Principles and Applications. WILEY VCH, 2002
- **J.A.O. SANCHEZ-PEDRONO et al.** *Anal. Chem. Acta,* 1986, vol. 182, 285
- **J.P. LÈRE-PORTE et al.** *Chem. Commun.,* 2002, vol. 24, 3020-3021
- **J.P. LÈRE-PORTE et al.** *Tet. Lett.,* 2001, vol. 42, 3073-3076